# EUROPEAN PATENT APPLICATION

(11) **EP 4 032 545 A1**
(43) Date of publication of application: **27.07.2022**
(21) Application number: 21153496.1
(22) Date of filing: 26.01.2021
(51) Int. Cl.: A61K 39/118, A61P 37/04, A61K 39/00

(54) **IMMUNOGENIC COMPOSITION AND VACCINE CONTAINING CHLAMYDIA SSP. SURFACE ANTIGENS AND ITS USE**

(71) Applicant: Medizinische Hochschule Hannover, 30625 Hannover (DE); Helmholtz-Zentrum für Infektionsforschung GmbH, 38124 Braunschweig (DE)
(72) Inventor: KLOS, Andreas, 30175 Hannover (DE); LAUDELEY, Robert, 31749 Auetal (DE); HEGEMANN, Johannes H., 54586 Schüller (DE); GUZMÁN, Carlos Alberto, 38304 Wolfenbüttel (DE); EBENSEN, Thomas, 30853 Langenhagen (DE); WINTGENS, Sebastian, 40589 Düsseldorf (DE)
(74) Representative: Gramm, Lins & Partner Patent- und Rechtsanwälte PartGmbB

(57) **Abstract**

The present invention relates in a first aspect to an immunogenic composition comprising at least three *Chlamydia ssp. surface* antigens selected from the group of PmpA, PmpD, PmpG, PmpH in combination with the antigen Ctad1. Further, the present invention relates to the immunogenic composition comprising CDN as adjuvant, in particular, c-diAMP. Moreover, a pharmaceutical composition comprising the immunogenic composition according to the present invention is provided as well as a vaccine comprising said immunogenic composition. The vaccine is particularly useful in eliciting an immune response against *Chlamydia ssp.* in an animal, in particular, for use in treating or preventing the infection by *Chlamydia ssp.* The vaccine, pharmaceutical composition or immunogenic composition may be administered mucosally, preferably is administered at least three times.

SEQUENCE LISTING

<110>  Medizinische Hochschule Hannover
       Helmholtz-Zentrum für Infektionsforschung GmbH

<120>  Immunogenic composition and vaccine containing Clamydia ssp.
       surface antigens and its use

<130>  3096-0062 EP-1

<160>  20

<170>  PatentIn version 3.5

<210>  1
<211>  613
<212>  PRT
<213>  Chlamydia trachomatis

<400>  1

Met Glu Leu Ala Ile Ser Gly His Lys Gln Gly Lys Asp Arg Asp Thr
1               5                   10                  15

Phe Thr Met Ile Ser Ser Cys Pro Glu Gly Thr Asn Tyr Ile Ile Asn
            20                  25                  30

Arg Lys Leu Ile Leu Ser Asp Phe Ser Leu Leu Asn Lys Val Ser Ser
        35                  40                  45

Gly Gly Ala Phe Arg Asn Leu Ala Gly Lys Ile Ser Phe Leu Gly Lys
    50                  55                  60

Asn Ser Ser Ala Ser Ile His Phe Lys His Ile Asn Ile Asn Gly Phe
65                  70                  75                  80

Gly Ala Gly Val Phe Ser Glu Ser Ser Ile Glu Phe Thr Asp Leu Arg
                85                  90                  95

Lys Leu Val Ala Phe Gly Ser Glu Ser Thr Gly Gly Ile Phe Thr Ala
            100                 105                 110

Lys Glu Asp Ile Ser Phe Lys Asn Asn His His Ile Ala Phe Arg Asn
        115                 120                 125

Asn Ile Thr Lys Gly Asn Gly Gly Val Ile Gln Leu Gln Gly Asp Met
    130                 135                 140

Lys Gly Ser Val Ser Phe Val Asp Gln Arg Gly Ala Ile Ile Phe Thr
145                 150                 155                 160

Asn Asn Gln Ala Val Thr Ser Ser Ser Met Lys His Ser Gly Arg Gly
                165                 170                 175

Gly Ala Ile Ser Gly Asp Phe Ala Gly Ser Arg Ile Leu Phe Leu Asn
            180                 185                 190

Asn Gln Gln Ile Thr Phe Glu Gly Asn Ser Ala Val His Gly Gly Ala
        195                 200                 205

Ile Tyr Asn Lys Asn Gly Leu Val Glu Phe Leu Gly Asn Ala Gly Pro
    210                 215                 220

Leu Ala Phe Lys Glu Asn Thr Thr Ile Ala Asn Gly Gly Ala Ile Tyr
225                 230                 235                 240

Thr Ser Asn Phe Lys Ala Asn Gln Gln Thr Ser Pro Ile Leu Phe Ser
                245                 250                 255

Gln Asn His Ala Asn Lys Lys Gly Gly Ala Ile Tyr Ala Gln Tyr Val
            260                 265                 270

Asn Leu Glu Gln Asn Gln Asp Thr Ile Arg Phe Glu Lys Asn Thr Ala
        275                 280                 285

Lys Glu Gly Gly Gly Ala Ile Thr Ser Ser Gln Cys Ser Ile Thr Ala
    290                 295                 300

His Asn Thr Ile Thr Phe Ser Asp Asn Ala Ala Gly Asp Leu Gly Gly
305                 310                 315                 320

Gly Ala Ile Leu Leu Glu Gly Lys Lys Pro Ser Leu Thr Leu Ile Ala
                325                 330                 335

His Ser Gly Asn Ile Ala Phe Ser Gly Asn Thr Met Leu His Ile Thr
            340                 345                 350

Lys Lys Ala Ser Leu Asp Arg His Asn Ser Ile Leu Ile Lys Glu Ala
        355                 360                 365

Pro Tyr Lys Ile Gln Leu Ala Ala Asn Lys Asn His Ser Ile His Phe
    370                 375                 380

Phe Asp Pro Val Met Ala Leu Ser Ala Ser Ser Ser Pro Ile Gln Ile
385                 390                 395                 400

Asn Ala Pro Glu Tyr Glu Thr Pro Phe Phe Ser Pro Lys Gly Met Ile
                405                 410                 415

Val Phe Ser Gly Ala Asn Leu Leu Asp Asp Ala Arg Glu Asp Val Ala

            420                 425                 430

Asn Arg Thr Ser Ile Phe Asn Gln Pro Val His Leu Tyr Asn Gly Thr
        435                 440                 445

Leu Ser Ile Glu Asn Gly Ala His Leu Ile Val Gln Ser Phe Lys Gln
    450                 455                 460

Thr Gly Gly Arg Ile Ser Leu Ser Pro Gly Ser Ser Leu Ala Leu Tyr
465                 470                 475                 480

Thr Met Asn Ser Phe Phe His Gly Asn Ile Ser Ser Lys Glu Pro Leu
                485                 490                 495

Glu Ile Asn Gly Leu Ser Phe Gly Val Asp Ile Ser Pro Ser Asn Leu
            500                 505                 510

Gln Ala Glu Ile Arg Ala Gly Asn Ala Pro Leu Arg Leu Ser Gly Ser
        515                 520                 525

Pro Ser Ile His Asp Pro Glu Gly Leu Phe Tyr Glu Asn Arg Asp Thr
    530                 535                 540

Ala Ala Ser Pro Tyr Gln Met Glu Ile Leu Leu Thr Ser Asp Lys Thr
545                 550                 555                 560

Val Asp Ile Ser Lys Phe Thr Thr Asp Ser Leu Val Thr Asn Lys Gln
                565                 570                 575

Ser Gly Phe Gln Gly Ala Trp His Phe Ser Trp Gln Pro Asn Thr Ile
            580                 585                 590

Asn Asn Thr Lys Gln Lys Ile Leu Arg Ala Ser Trp Leu Pro Thr Gly
        595                 600                 605

Glu Tyr Val Leu Glu
    610

<210> 2
<211> 585
<212> PRT
<213> Chlamydia tachomatis

<400> 2

Leu His Ala Gly Gly Gln Ser Val Asn Glu Leu Val Tyr Val Gly Pro
1               5                   10                  15

Gln Ala Val Leu Leu Leu Asp Gln Ile Arg Asp Leu Phe Val Gly Ser

20                  25                  30

Lys Asp Ser Gln Ala Glu Gly Gln Tyr Arg Leu Ile Val Gly Asp Pro
        35                  40                  45

Ser Ser Phe Gln Glu Lys Asp Ala Asp Thr Leu Pro Gly Lys Val Glu
    50                  55                  60

Gln Ser Thr Leu Phe Ser Val Thr Asn Pro Val Val Phe Gln Gly Val
65                  70                  75                  80

Asp Gln Gln Asp Gln Val Ser Ser Gln Gly Leu Ile Cys Ser Phe Thr
                85                  90                  95

Ser Ser Asn Leu Asp Ser Pro Arg Asp Gly Glu Ser Phe Leu Gly Ile
            100                 105                 110

Ala Phe Val Gly Asp Ser Ser Lys Ala Gly Ile Thr Leu Thr Asp Val
        115                 120                 125

Lys Ala Ser Leu Ser Gly Ala Ala Leu Tyr Ser Thr Glu Asp Leu Ile
    130                 135                 140

Phe Glu Lys Ile Lys Gly Gly Leu Glu Phe Ala Ser Cys Ser Ser Leu
145                 150                 155                 160

Glu Gln Gly Gly Ala Cys Ala Ala Gln Ser Ile Leu Ile His Asp Cys
                165                 170                 175

Gln Gly Leu Gln Val Lys His Cys Thr Thr Ala Val Asn Ala Glu Gly
            180                 185                 190

Ser Ser Ala Asn Asp His Leu Gly Phe Gly Gly Gly Ala Phe Phe Val
        195                 200                 205

Thr Gly Ser Leu Ser Gly Glu Lys Ser Leu Tyr Met Pro Ala Gly Asp
    210                 215                 220

Met Val Val Ala Asn Cys Asp Gly Ala Ile Ser Phe Glu Gly Asn Ser
225                 230                 235                 240

Ala Asn Phe Ala Asn Gly Gly Ala Ile Ala Ala Ser Gly Lys Val Leu
                245                 250                 255

Phe Val Ala Asn Asp Lys Lys Thr Ser Phe Ile Glu Asn Arg Ala Leu
            260                 265                 270

Ser Gly Gly Ala Ile Ala Ala Ser Ser Asp Ile Ala Phe Gln Asn Cys
        275                 280                 285

Ala Glu Leu Val Phe Lys Gly Asn Cys Ala Ile Gly Thr Glu Asp Lys
    290                 295                 300

Gly Ser Leu Gly Gly Gly Ala Ile Ser Ser Leu Gly Thr Val Leu Leu
305                 310                 315                 320

Gln Gly Asn His Gly Ile Thr Cys Asp Lys Asn Glu Ser Ala Ser Gln
                325                 330                 335

Gly Gly Ala Ile Phe Gly Lys Asn Cys Gln Ile Ser Asp Asn Glu Gly
            340                 345                 350

Pro Val Val Phe Arg Asp Ser Thr Ala Cys Leu Gly Gly Gly Ala Ile
        355                 360                 365

Ala Ala Gln Glu Ile Val Ser Ile Gln Asn Asn Gln Ala Gly Ile Ser
    370                 375                 380

Phe Glu Gly Gly Lys Ala Ser Phe Gly Gly Gly Ile Ala Cys Glu Ser
385                 390                 395                 400

Phe Ser Ser Ala Gly Gly Ala Ser Val Leu Gly Ala Ile Asp Ile Ser
                405                 410                 415

Lys Asn Leu Gly Ala Ile Ser Phe Ser Arg Thr Leu Cys Thr Thr Ser
            420                 425                 430

Asp Leu Gly Gln Met Glu Tyr Gln Gly Gly Gly Ala Leu Phe Gly Glu
        435                 440                 445

Asn Ile Ser Leu Ser Glu Asn Ala Gly Val Leu Thr Phe Lys Asp Asn
    450                 455                 460

Ile Val Lys Thr Phe Ala Ser Asn Gly Lys Ile Leu Gly Gly Gly Ala
465                 470                 475                 480

Ile Leu Ala Thr Gly Lys Val Glu Ile Thr Asn Asn Ser Gly Gly Ile
                485                 490                 495

Ser Phe Thr Gly Asn Ala Arg Ala Pro Gln Ala Leu Pro Thr Gln Glu
            500                 505                 510

Glu Phe Pro Leu Phe Ser Lys Lys Glu Gly Arg Pro Leu Ser Ser Gly
        515                 520                 525

Tyr Ser Gly Gly Gly Ala Ile Leu Gly Arg Glu Val Ala Ile Leu His
    530                 535                 540

Asn Ala Ala Val Val Phe Glu Gln Asn Arg Leu Gln Cys Ser Glu Glu
545                 550                 555                 560

Glu Ala Thr Leu Leu Gly Cys Cys Gly Gly Gly Ala Val His Gly Met
                565                 570                 575

Asp Ser Thr Ser Ile Val Gly Asn Ser
            580                 585

<210>  3
<211>  645
<212>  PRT
<213>  Chlamydia trachomatis

<400>  3

Glu Ile Met Val Pro Gln Gly Ile Tyr Asp Gly Glu Thr Leu Thr Val
1               5                   10                  15

Ser Phe Pro Tyr Thr Val Ile Gly Asp Pro Ser Gly Thr Thr Val Phe
            20                  25                  30

Ser Ala Gly Glu Leu Thr Leu Lys Asn Leu Asp Asn Ser Ile Ala Ala
        35                  40                  45

Leu Pro Leu Ser Cys Phe Gly Asn Leu Leu Gly Ser Phe Thr Val Leu
    50                  55                  60

Gly Arg Gly His Ser Leu Thr Phe Glu Asn Ile Arg Thr Ser Thr Asn
65                  70                  75                  80

Gly Ala Ala Leu Ser Asp Ser Ala Asn Ser Gly Leu Phe Thr Ile Glu
                85                  90                  95

Gly Phe Lys Glu Leu Ser Phe Ser Asn Cys Asn Ser Leu Leu Ala Val
            100                 105                 110

Leu Pro Ala Ala Thr Thr Asn Asn Gly Ser Gln Thr Pro Thr Thr Thr
        115                 120                 125

Ser Thr Pro Ser Asn Gly Thr Ile Tyr Ser Lys Thr Asp Leu Leu Leu
    130                 135                 140

Leu Asn Asn Glu Lys Phe Ser Phe Tyr Ser Asn Leu Val Ser Gly Asp
145                 150                 155                 160

Gly Gly Ala Ile Asp Ala Lys Ser Leu Thr Val Gln Gly Ile Ser Lys
                165                 170                 175

Leu Cys Val Phe Gln Glu Asn Thr Ala Gln Ala Asp Gly Gly Ala Cys
            180                 185                 190

Gln Val Val Thr Ser Phe Ser Ala Met Ala Asn Glu Ala Pro Ile Ala
        195                 200                 205

Phe Val Ala Asn Val Ala Gly Val Arg Gly Gly Gly Ile Ala Ala Val
    210                 215                 220

Gln Asp Gly Gln Gln Gly Val Ser Ser Ser Thr Ser Thr Glu Asp Pro
225                 230                 235                 240

Val Val Ser Phe Ser Arg Asn Thr Ala Val Glu Phe Asp Gly Asn Val
                245                 250                 255

Ala Arg Val Gly Gly Gly Ile Tyr Ser Tyr Gly Asn Val Ala Phe Leu
            260                 265                 270

Asn Asn Gly Lys Thr Leu Phe Leu Asn Asn Val Ala Ser Pro Val Tyr
        275                 280                 285

Ile Ala Ala Glu Gln Pro Thr Asn Gly Gln Ala Ser Asn Thr Ser Asp
    290                 295                 300

Asn Tyr Gly Asp Gly Gly Ala Ile Phe Cys Lys Asn Gly Ala Gln Ala
305                 310                 315                 320

Ala Gly Ser Asn Asn Ser Gly Ser Val Ser Phe Asp Gly Glu Gly Val
                325                 330                 335

Val Phe Phe Ser Ser Asn Val Ala Ala Gly Lys Gly Gly Ala Ile Tyr
            340                 345                 350

Ala Lys Lys Leu Ser Val Ala Asn Cys Gly Pro Val Gln Phe Leu Gly
        355                 360                 365

Asn Ile Ala Asn Asp Gly Gly Ala Ile Tyr Leu Gly Glu Ser Gly Glu
    370                 375                 380

Leu Ser Leu Ser Ala Asp Tyr Gly Asp Ile Ile Phe Asp Gly Asn Leu
385                 390                 395                 400

Lys Arg Thr Ala Lys Glu Asn Ala Ala Asp Val Asn Gly Val Thr Val
                405                 410                 415

Ser Ser Gln Ala Ile Ser Met Gly Ser Gly Gly Lys Ile Thr Thr Leu
            420                 425                 430

Arg Ala Lys Ala Gly His Gln Ile Leu Phe Asn Asp Pro Ile Glu Met
        435                 440                 445

Ala Asn Gly Asn Asn Gln Pro Ala Gln Ser Ser Glu Pro Leu Lys Ile
    450                 455                 460

Asn Asp Gly Glu Gly Tyr Thr Gly Asp Ile Val Phe Ala Asn Gly Asn
465                 470                 475                 480

Ser Thr Leu Tyr Gln Asn Val Thr Ile Glu Gln Gly Arg Ile Val Leu
                485                 490                 495

Arg Glu Lys Ala Lys Leu Ser Val Asn Ser Leu Ser Gln Thr Gly Gly
            500                 505                 510

Ser Leu Tyr Met Glu Ala Gly Ser Thr Leu Asp Phe Val Thr Pro Gln
        515                 520                 525

Pro Pro Gln Gln Pro Pro Ala Ala Asn Gln Leu Ile Thr Leu Ser Asn
    530                 535                 540

Leu His Leu Ser Leu Ser Ser Leu Leu Ala Asn Asn Ala Val Thr Asn
545                 550                 555                 560

Pro Pro Thr Asn Pro Pro Ala Gln Asp Ser His Pro Ala Val Ile Gly
                565                 570                 575

Ser Thr Thr Ala Gly Ser Val Thr Ile Ser Gly Pro Ile Phe Phe Glu
            580                 585                 590

Asp Leu Asp Asp Thr Ala Tyr Asp Arg Tyr Asp Trp Leu Gly Ser Asn
        595                 600                 605

Gln Lys Ile Asp Val Leu Lys Leu Gln Leu Gly Thr Gln Pro Pro Ala
    610                 615                 620

Asn Ala Pro Ser Asp Leu Thr Leu Gly Asn Glu Met Pro Lys Tyr Gly
625                 630                 635                 640

Tyr Gln Gly Ser Trp
                645

<210> 4

<211>  665
<212>  PRT
<213>  Chlamydia trachomatis

<400>  4

Ser Pro Gln Val Leu Thr Pro Asn Val Thr Thr Pro Phe Lys Gly Asp
1               5                   10                  15

Asp Val Tyr Leu Asn Gly Asp Cys Ala Phe Val Asn Val Tyr Ala Gly
            20                  25                  30

Ala Glu Asn Gly Ser Ile Ile Ser Ala Asn Gly Asp Asn Leu Thr Ile
        35                  40                  45

Thr Gly Gln Asn His Thr Leu Ser Phe Thr Asp Ser Gln Gly Pro Val
    50                  55                  60

Leu Gln Asn Tyr Ala Phe Ile Ser Ala Gly Glu Thr Leu Thr Leu Lys
65                  70                  75                  80

Asp Phe Ser Ser Leu Met Phe Ser Lys Asn Val Ser Cys Gly Glu Lys
                85                  90                  95

Gly Met Ile Ser Gly Lys Thr Val Ser Ile Ser Gly Ala Gly Glu Val
            100                 105                 110

Ile Phe Trp Asp Asn Ser Val Gly Tyr Ser Pro Leu Ser Thr Val Pro
        115                 120                 125

Ala Ser Thr Pro Thr Pro Pro Ala Pro Ala Pro Ala Ala Ser Ser Ser
    130                 135                 140

Leu Ser Pro Thr Val Ser Asp Ala Arg Lys Gly Ser Ile Phe Ser Val
145                 150                 155                 160

Glu Thr Ser Leu Glu Ile Ser Gly Val Lys Lys Gly Val Met Phe Asp
                165                 170                 175

Asn Asn Ala Gly Asn Phe Gly Thr Val Phe Arg Gly Asn Ser Asn Asn
            180                 185                 190

Asn Ala Gly Ser Gly Gly Ser Gly Ser Ala Thr Thr Pro Ser Phe Thr
        195                 200                 205

Val Lys Asn Cys Lys Gly Lys Val Ser Phe Thr Asp Asn Val Ala Ser
    210                 215                 220

Cys Gly Gly Gly Val Val Tyr Lys Gly Thr Val Leu Phe Lys Asp Asn

225                 230                 235                 240

Glu Gly Gly Ile Phe Phe Arg Gly Asn Thr Ala Tyr Asp Asp Leu Gly
                245                 250                 255

Ile Leu Ala Ala Thr Ser Arg Asp Gln Asn Thr Glu Thr Gly Gly Gly
            260                 265                 270

Gly Gly Val Ile Cys Ser Pro Asp Asp Ser Val Lys Phe Glu Gly Asn
        275                 280                 285

Lys Gly Ser Ile Val Phe Asp Tyr Asn Phe Ala Lys Gly Arg Gly Gly
    290                 295                 300

Ser Ile Leu Thr Lys Glu Phe Ser Leu Val Ala Asp Asp Ser Val Val
305                 310                 315                 320

Phe Ser Asn Asn Thr Ala Glu Lys Gly Gly Gly Ala Ile Tyr Ala Pro
                325                 330                 335

Thr Ile Asp Ile Ser Thr Asn Gly Gly Ser Ile Leu Phe Glu Arg Asn
            340                 345                 350

Arg Ala Ala Glu Gly Gly Ala Ile Cys Val Ser Glu Ala Ser Ser Gly
        355                 360                 365

Ser Thr Gly Asn Leu Thr Leu Ser Ala Ser Asp Gly Asp Ile Val Phe
    370                 375                 380

Ser Gly Asn Met Thr Ser Asp Arg Pro Gly Glu Arg Ser Ala Ala Arg
385                 390                 395                 400

Ile Leu Ser Asp Gly Thr Thr Val Ser Leu Asn Ala Ser Gly Leu Ser
                405                 410                 415

Lys Leu Ile Phe Tyr Asp Pro Val Val Gln Asn Asn Ser Ala Ala Gly
            420                 425                 430

Ala Ser Thr Pro Ser Pro Ser Ser Ser Ser Met Pro Gly Ala Val Thr
        435                 440                 445

Ile Asn Gln Ser Gly Asn Gly Ser Val Ile Phe Thr Ala Glu Ser Leu
    450                 455                 460

Thr Pro Ser Glu Lys Leu Gln Val Leu Asn Ser Thr Ser Asn Phe Pro
465                 470                 475                 480

Gly Ala Leu Thr Val Ser Gly Gly Glu Leu Val Val Thr Glu Gly Ala
                    485                 490                 495

Thr Leu Thr Thr Gly Thr Ile Thr Ala Thr Ser Gly Arg Val Thr Leu
            500                 505                 510

Gly Ser Gly Ala Ser Leu Ser Ala Val Ala Gly Ala Ala Asn Asn Asn
        515                 520                 525

Tyr Thr Cys Thr Val Ser Lys Leu Gly Ile Asp Leu Glu Ser Phe Leu
    530                 535                 540

Thr Pro Asn Tyr Lys Thr Ala Ile Leu Gly Ala Asp Gly Thr Val Thr
545                 550                 555                 560

Val Asn Ser Gly Ser Thr Leu Asp Leu Val Met Glu Ser Glu Ala Glu
                565                 570                 575

Val Tyr Asp Asn Pro Leu Phe Val Gly Ser Leu Thr Ile Pro Phe Val
            580                 585                 590

Thr Leu Ser Ser Ser Ser Ala Ser Asn Gly Val Thr Lys Asn Ser Val
        595                 600                 605

Thr Ile Asn Asp Ala Asp Ala Ala His Tyr Gly Tyr Gln Gly Ser Trp
    610                 615                 620

Ser Ala Asp Trp Thr Lys Pro Pro Leu Ala Pro Asp Ala Lys Gly Met
625                 630                 635                 640

Val Pro Pro Asn Thr Asn Asn Thr Leu Tyr Leu Thr Trp Arg Pro Ala
                645                 650                 655

Ser Asn Tyr Gly Glu Tyr Arg Leu Asp
            660                 665

<210>  5
<211>  433
<212>  PRT
<213>  Chlamydia Trachomatis

<400>  5

Met Leu Ile Phe Ala Leu Ser Phe Gly Ala Asp Ala Cys Leu Cys Ala
1               5                   10                  15

Ala Asp Leu Ser Lys Ala Lys Val Glu Ala Ser Val Gly Asp Arg Ala
            20                  25                  30

Ala Phe Ser Pro Phe Thr Gly Glu Ile Lys Gly Asn Arg Val Arg Leu
        35                  40                  45

Arg Leu Ala Pro His Thr Asp Ser Phe Ile Ile Lys Glu Leu Ser Lys
    50                  55                  60

Gly Asp Cys Leu Ala Val Leu Gly Glu Ser Lys Asp Tyr Tyr Val Val
65                  70                  75                  80

Ala Ala Pro Glu Gly Val Arg Gly Tyr Val Phe Arg Thr Phe Val Leu
                85                  90                  95

Asp Asn Val Ile Glu Gly Glu Lys Val Asn Val Arg Leu Glu Pro Ser
            100                 105                 110

Thr Ser Ala Pro Ile Leu Ala Arg Leu Ser Lys Gly Thr Val Val Lys
        115                 120                 125

Thr Leu Gly Ala Ala Gln Gly Lys Trp Ile Glu Ile Ala Leu Pro Lys
    130                 135                 140

Gln Cys Val Phe Tyr Val Ala Lys Asn Phe Val Lys Asn Val Gly Ala
145                 150                 155                 160

Leu Asp Leu Tyr Asn Gln Lys Glu Gly Gln Lys Lys Leu Ala Leu Asp
                165                 170                 175

Leu Leu Ser Ser Ala Met Asp Phe Ala Asp Ala Glu Leu Gln Lys Lys
            180                 185                 190

Ile Glu Asp Ile Asp Leu Asp Ala Ile Tyr Lys Lys Met Asn Leu Ala
        195                 200                 205

Gln Ser Glu Glu Phe Lys Asp Val Pro Gly Leu Gln Ser Leu Val Gln
    210                 215                 220

Lys Ala Leu Glu Arg Val Gln Glu Ala Phe Leu Ala Lys Ser Leu Glu
225                 230                 235                 240

Lys Ser Ser Val Lys Val Pro Glu Ile Arg His Lys Val Leu Glu Glu
                245                 250                 255

Ile Ala Val Val Ser Pro Ala Val Glu Glu Thr Pro Val Val Thr Lys
            260                 265                 270

Thr Glu Glu Gln Lys Val Thr Thr Val Pro Val Pro Ala Pro Ala Val
        275                 280                 285

Val Thr Glu Pro Ala Gln Asp Leu Ser Ser Val Lys Gly Ser Leu Leu
    290                 295                 300

Ser His Tyr Ile Arg Lys Lys Gly Phe Val Lys Ala Ser Pro Val Ile
305                 310                 315                 320

Glu Gly Arg Glu Ser Phe Glu Arg Ser Leu Phe Ala Val Trp Leu Ser
                325                 330                 335

Leu Gln Pro Glu Glu Ile Arg His Gln Leu Thr Met Glu Ser Phe Tyr
            340                 345                 350

Arg Asp Glu Gln Lys Lys Lys Arg Val Leu Thr Gly Glu Leu Glu Val
        355                 360                 365

Tyr Pro His Ile Val Lys Asn Asn Pro Gly Asp Tyr Leu Leu Lys Asn
    370                 375                 380

Gly Glu Asp Val Val Ala Phe Val Tyr Ala Thr Ser Ile Asp Leu Ser
385                 390                 395                 400

Lys Trp Leu Gly Lys Ser Val Val Leu Glu Cys Val Ser Arg Pro Asn
                405                 410                 415

Asn His Phe Ala Phe Pro Ala Tyr Ile Val Leu Ser Val Lys Glu Gly
            420                 425                 430

Ala

<210>  6
<211>  433
<212>  PRT
<213>  Chlamydia Trachomatis

<400>  6

Met Leu Ile Phe Ala Leu Ser Phe Gly Ala Asp Ala Cys Leu Cys Ala
1               5                   10                  15

Ala Asp Leu Ser Lys Ala Lys Val Glu Ala Ser Val Gly Asp Arg Ala
            20                  25                  30

Ala Phe Ser Pro Phe Thr Gly Glu Ile Lys Gly Asn Arg Val Arg Leu
        35                  40                  45

Arg Leu Ala Pro His Thr Asp Ser Phe Ile Ile Lys Glu Leu Ser Lys
    50                  55                  60

Gly Asp Cys Leu Ala Val Leu Gly Glu Ser Lys Asp Tyr Tyr Val Val
65                  70                  75                  80

Ala Ala Pro Glu Gly Val Arg Gly Tyr Val Phe Arg Thr Phe Val Leu
                85                  90                  95

Asp Asn Val Ile Glu Gly Glu Lys Val Asn Val Arg Leu Glu Pro Ser
            100                 105                 110

Thr Ser Ala Pro Ile Leu Ala Arg Leu Ser Lys Gly Thr Val Val Lys
        115                 120                 125

Thr Leu Gly Ala Ala Gln Gly Lys Trp Ile Glu Ile Ala Leu Pro Lys
    130                 135                 140

Gln Cys Val Phe Tyr Val Ala Lys Asn Phe Val Lys Asn Val Gly Ala
145                 150                 155                 160

Leu Asp Leu Tyr Asn Gln Lys Glu Gly Gln Lys Lys Leu Ala Leu Asp
                165                 170                 175

Leu Leu Ser Ser Ala Met Asp Phe Ala Asp Ala Glu Leu Gln Lys Lys
            180                 185                 190

Ile Glu Asp Ile Asp Leu Asp Ala Ile Tyr Lys Lys Met Asn Leu Ala
        195                 200                 205

Gln Ser Glu Glu Phe Lys Asp Val Pro Gly Leu Gln Ser Leu Val Gln
    210                 215                 220

Lys Ala Leu Glu Arg Val Gln Glu Ala Phe Leu Ala Lys Ser Leu Glu
225                 230                 235                 240

Lys Ser Ser Val Lys Val Pro Glu Ile Arg His Lys Val Leu Glu Glu
                245                 250                 255

Ile Ala Val Val Ser Pro Ala Val Glu Glu Thr Pro Val Val Thr Lys
            260                 265                 270

Thr Glu Glu Gln Lys Val Thr Thr Val Pro Val Pro Ala Pro Ala Val
        275                 280                 285

Val Thr Glu Pro Ala Gln Asp Leu Ser Ser Val Lys Gly Ser Leu Leu
    290                 295                 300

Ser His Tyr Ile Arg Lys Lys Gly Phe Val Lys Ala Ser Pro Val Ile
305                 310                 315                 320

Glu Gly Arg Glu Ser Phe Glu Arg Ser Leu Phe Ala Val Trp Leu Ser
                325                 330                 335

Leu Gln Pro Glu Glu Ile Arg His Gln Leu Thr Met Glu Ser Phe Tyr
            340                 345                 350

Arg Asp Glu Gln Lys Lys Lys Arg Val Leu Thr Gly Glu Leu Glu Val
        355                 360                 365

Tyr Pro His Ile Val Lys Asn Asn Pro Gly Asp Tyr Leu Leu Lys Asn
    370                 375                 380

Gly Glu Asp Val Val Ala Phe Val Tyr Ala Thr Ser Ile Asp Leu Ser
385                 390                 395                 400

Lys Trp Leu Gly Lys Ser Val Val Leu Glu Cys Val Ser Arg Pro Asn
                405                 410                 415

Asn His Phe Ala Phe Pro Ala Tyr Ile Val Leu Ser Val Lys Glu Gly
            420                 425                 430

Ala

<210>  7
<211>  888
<212>  PRT
<213>  Chlamydia Trachomatis

<400>  7

Leu His Ala Gly Gly Gln Ser Val Asn Glu Leu Val Tyr Val Gly Pro
1               5                   10                  15

Gln Ala Val Leu Leu Leu Asp Gln Ile Arg Asp Leu Phe Val Gly Ser
            20                  25                  30

Lys Asp Ser Gln Ala Glu Gly Gln Tyr Arg Leu Ile Val Gly Asp Pro
        35                  40                  45

Ser Ser Phe Gln Glu Lys Asp Ala Asp Thr Leu Pro Gly Lys Val Glu
    50                  55                  60

Gln Ser Thr Leu Phe Ser Val Thr Asn Pro Val Val Phe Gln Gly Val
65                  70                  75                  80

Asp Gln Gln Asp Gln Val Ser Ser Gln Gly Leu Ile Cys Ser Phe Thr
                85                  90                  95

Ser Ser Asn Leu Asp Ser Pro Arg Asp Gly Glu Ser Phe Leu Gly Ile
            100                 105                 110

Ala Phe Val Gly Asp Ser Ser Lys Ala Gly Ile Thr Leu Thr Asp Val
        115                 120                 125

Lys Ala Ser Leu Ser Gly Ala Ala Leu Tyr Ser Thr Glu Asp Leu Ile
    130                 135                 140

Phe Glu Lys Ile Lys Gly Gly Leu Glu Phe Ala Ser Cys Ser Ser Leu
145                 150                 155                 160

Glu Gln Gly Gly Ala Cys Ala Ala Gln Ser Ile Leu Ile His Asp Cys
                165                 170                 175

Gln Gly Leu Gln Val Lys His Cys Thr Thr Ala Val Asn Ala Glu Gly
            180                 185                 190

Ser Ser Ala Asn Asp His Leu Gly Phe Gly Gly Gly Ala Phe Phe Val
        195                 200                 205

Thr Gly Ser Leu Ser Gly Glu Lys Ser Leu Tyr Met Pro Ala Gly Asp
    210                 215                 220

Met Val Val Ala Asn Cys Asp Gly Ala Ile Ser Phe Glu Gly Asn Ser
225                 230                 235                 240

Ala Asn Phe Ala Asn Gly Gly Ala Ile Ala Ala Ser Gly Lys Val Leu
                245                 250                 255

Phe Val Ala Asn Asp Lys Lys Thr Ser Phe Ile Glu Asn Arg Ala Leu
            260                 265                 270

Ser Gly Gly Ala Ile Ala Ala Ser Ser Asp Ile Ala Phe Gln Asn Cys
        275                 280                 285

Ala Glu Leu Val Phe Lys Gly Asn Cys Ala Ile Gly Thr Glu Asp Lys
    290                 295                 300

Gly Ser Leu Gly Gly Gly Ala Ile Ser Ser Leu Gly Thr Val Leu Leu
305                 310                 315                 320

Gln Gly Asn Tyr Gly Ile Thr Cys Asp Lys Asn Glu Ser Ala Ser Gln
                325                 330                 335

Gly Gly Ala Ile Phe Gly Lys Asn Cys Gln Ile Ser Asp Asn Glu Gly

            340                 345                 350

Pro Val Val Phe Arg Asp Ser Thr Ala Cys Leu Gly Gly Gly Ala Ile
        355                 360                 365

Ala Ala Gln Glu Ile Val Ser Ile Gln Asn Asn Gln Ala Gly Ile Ser
    370                 375                 380

Phe Glu Gly Gly Lys Ala Ser Phe Gly Gly Gly Ile Ala Cys Gly Ser
385                 390                 395                 400

Phe Ser Ser Ala Gly Gly Ala Ser Val Leu Gly Thr Ile Asp Ile Ser
                405                 410                 415

Lys Asn Leu Gly Ala Ile Ser Phe Ser Arg Thr Leu Cys Thr Thr Ser
            420                 425                 430

Asp Leu Gly Gln Met Glu Tyr Gln Gly Gly Gly Ala Leu Phe Gly Glu
        435                 440                 445

Asn Ile Ser Leu Ser Glu Asn Ala Gly Val Leu Thr Phe Lys Asp Asn
    450                 455                 460

Ile Val Lys Thr Phe Ala Ser Asn Gly Lys Ile Leu Gly Gly Gly Ala
465                 470                 475                 480

Ile Leu Ala Thr Gly Lys Val Glu Ile Thr Asn Asn Ser Gly Gly Ile
                485                 490                 495

Ser Phe Thr Gly Asn Ala Arg Ala Pro Gln Ala Leu Pro Thr Gln Glu
            500                 505                 510

Glu Phe Pro Leu Phe Ser Lys Lys Glu Gly Arg Pro Leu Ser Ser Gly
        515                 520                 525

Tyr Ser Gly Gly Gly Ala Ile Leu Gly Arg Glu Val Ala Ile Leu His
    530                 535                 540

Asn Ala Ala Val Val Phe Glu Gln Asn Arg Leu Gln Cys Ser Glu Glu
545                 550                 555                 560

Glu Ala Thr Leu Leu Gly Cys Cys Gly Gly Gly Ala Val His Gly Met
                565                 570                 575

Asp Ser Thr Ser Ile Val Gly Asn Ser Ser Val Arg Phe Gly Asn Asn
            580                 585                 590

Tyr Ala Met Gly Gln Gly Val Ser Gly Gly Ala Leu Leu Ser Lys Thr
        595                 600                 605

Val Gln Leu Ala Gly Asn Gly Ser Val Asp Phe Ser Arg Asn Ile Ala
    610                 615                 620

Ser Leu Gly Gly Gly Ala Leu Gln Ala Ser Glu Gly Asn Cys Glu Leu
625                 630                 635                 640

Val Asp Asn Gly Tyr Val Leu Phe Arg Asp Asn Arg Gly Arg Val Tyr
                645                 650                 655

Gly Gly Ala Ile Ser Cys Leu Arg Gly Asp Val Val Ile Ser Gly Asn
            660                 665                 670

Lys Gly Arg Val Glu Phe Lys Asp Asn Ile Ala Thr Arg Leu Tyr Val
        675                 680                 685

Glu Glu Thr Val Glu Lys Val Glu Glu Val Glu Pro Ala Pro Glu Gln
    690                 695                 700

Lys Asp Asn Asn Glu Leu Ser Phe Leu Gly Ser Ala Glu Gln Ser Phe
705                 710                 715                 720

Ile Thr Ala Ala Asn Gln Ala Leu Phe Val Ser Glu Asp Gly Asp Leu
                725                 730                 735

Ser Pro Glu Ser Ser Ile Ser Ser Glu Glu Leu Val Lys Arg Arg Glu
            740                 745                 750

Cys Ala Gly Gly Ala Ile Phe Ala Lys Arg Val Arg Ile Val Asp Asn
        755                 760                 765

Gln Glu Ala Val Val Phe Ser Asn Asn Phe Ser Asp Ile Tyr Gly Gly
    770                 775                 780

Ala Ile Phe Thr Gly Ser Leu Arg Glu Glu Asp Lys Leu Asp Gly Gln
785                 790                 795                 800

Ile Pro Glu Val Leu Ile Ser Gly Asn Ala Gly Asp Val Val Phe Ser
                805                 810                 815

Gly Asn Ser Ser Lys His Asp Glu His Leu Pro His Thr Gly Gly Gly
            820                 825                 830

Ala Ile Cys Thr Gln Asn Leu Thr Ile Ser Gln Asn Thr Gly Asn Val
        835                 840                 845

Leu Phe Tyr Asn Asn Val Ala Cys Ser Gly Gly Ala Val Arg Ile Glu
    850                 855                 860

Asp His Gly Asn Val Leu Leu Glu Ala Phe Gly Gly Asp Ile Val Phe
865                 870                 875                 880

Lys Gly Asn Ser Ser Thr Arg Asx
                885

<210>  8
<211>  645
<212>  PRT
<213>  Chlamydia Trachomatis

<400>  8

Glu Ile Met Val Pro Gln Gly Ile Tyr Asp Gly Glu Thr Leu Thr Val
1               5                   10                  15

Ser Phe Pro Tyr Thr Val Ile Gly Asp Pro Ser Gly Thr Thr Val Phe
            20                  25                  30

Ser Ala Gly Glu Leu Thr Leu Lys Asn Leu Asp Asn Ser Ile Ala Ala
        35                  40                  45

Leu Pro Leu Ser Cys Phe Gly Asn Leu Leu Gly Ser Phe Thr Val Leu
    50                  55                  60

Gly Arg Gly His Ser Leu Thr Phe Glu Asn Ile Arg Thr Ser Thr Asn
65                  70                  75                  80

Gly Ala Ala Leu Ser Asp Ser Ala Asn Ser Gly Leu Phe Thr Ile Glu
                85                  90                  95

Gly Phe Lys Glu Leu Ser Phe Ser Asn Cys Asn Ser Leu Leu Ala Val
            100                 105                 110

Leu Pro Ala Ala Thr Thr Asn Asn Gly Ser Gln Thr Pro Thr Thr Thr
        115                 120                 125

Ser Thr Pro Ser Asn Gly Thr Ile Tyr Ser Lys Thr Asp Leu Leu Leu
    130                 135                 140

Leu Asn Asn Glu Lys Phe Ser Phe Tyr Ser Asn Leu Val Ser Gly Asp
145                 150                 155                 160

Gly Gly Ala Ile Asp Ala Lys Ser Leu Thr Val Gln Gly Ile Ser Lys
                165                 170                 175

Leu Cys Val Phe Gln Glu Asn Thr Ala Gln Ala Asp Gly Gly Ala Cys
        180                 185                 190

Gln Val Val Thr Ser Phe Ser Ala Met Ala Asn Glu Ala Pro Ile Ala
    195                 200                 205

Phe Val Ala Asn Val Ala Gly Val Arg Gly Gly Gly Ile Ala Ala Val
    210                 215                 220

Gln Asp Gly Gln Gln Gly Val Ser Ser Ser Thr Ser Thr Glu Asp Pro
225                 230                 235                 240

Val Val Ser Phe Ser Arg Asn Thr Ala Val Glu Phe Asp Gly Asn Val
                245                 250                 255

Ala Arg Val Gly Gly Gly Ile Tyr Ser Tyr Gly Asn Val Ala Phe Leu
            260                 265                 270

Asn Asn Gly Lys Thr Leu Phe Leu Asn Asn Val Ala Ser Pro Val Tyr
        275                 280                 285

Ile Ala Ala Glu Gln Pro Thr Asn Gly Gln Ala Ser Asn Thr Ser Asp
    290                 295                 300

Asn Tyr Gly Asp Gly Gly Ala Ile Phe Cys Lys Asn Gly Ala Gln Ala
305                 310                 315                 320

Ala Gly Ser Asn Asn Ser Gly Ser Val Ser Phe Asp Gly Glu Gly Val
                325                 330                 335

Val Phe Phe Ser Ser Asn Val Ala Ala Gly Lys Gly Gly Ala Ile Tyr
            340                 345                 350

Ala Lys Lys Leu Ser Val Ala Asn Cys Gly Pro Val Gln Phe Leu Gly
        355                 360                 365

Asn Ile Ala Asn Asp Gly Gly Ala Ile Tyr Leu Gly Glu Ser Gly Glu
    370                 375                 380

Leu Ser Leu Ser Ala Asp Tyr Gly Asp Met Ile Phe Asp Gly Asn Leu
385                 390                 395                 400

Lys Arg Thr Ala Lys Glu Asn Ala Ala Asp Val Asn Gly Val Thr Val
                405                 410                 415

Ser Ser Gln Ala Ile Ser Met Gly Ser Gly Gly Lys Ile Thr Thr Leu
            420                 425                 430

Arg Ala Lys Ala Gly His Gln Ile Leu Phe Asn Asp Pro Ile Glu Met
        435                 440                 445

Ala Asn Gly Asn Asn Gln Pro Ala Gln Ser Ser Glu Pro Leu Lys Ile
    450                 455                 460

Asn Asp Gly Glu Gly Tyr Thr Gly Asp Ile Val Phe Ala Asn Gly Asn
465                 470                 475                 480

Ser Thr Leu Tyr Gln Asn Val Thr Ile Glu Gln Gly Arg Ile Val Leu
                485                 490                 495

Arg Glu Lys Ala Lys Leu Ser Val Asn Ser Leu Ser Gln Thr Gly Gly
            500                 505                 510

Ser Leu Tyr Met Glu Ala Gly Ser Thr Leu Asp Phe Val Thr Pro Gln
        515                 520                 525

Pro Pro Gln Gln Pro Pro Ala Ala Asn Gln Leu Ile Thr Leu Ser Asn
    530                 535                 540

Leu His Leu Ser Leu Ser Ser Leu Leu Ala Asn Asn Ala Val Thr Asn
545                 550                 555                 560

Pro Pro Thr Asn Pro Pro Ala Gln Asp Ser His Pro Ala Val Ile Gly
                565                 570                 575

Ser Thr Thr Ala Gly Ser Val Thr Ile Ser Gly Pro Ile Phe Phe Glu
            580                 585                 590

Asp Leu Asp Asp Thr Ala Tyr Asp Arg Tyr Asp Trp Leu Gly Ser Asn
        595                 600                 605

Gln Lys Ile Asp Val Leu Lys Leu Gln Leu Gly Thr Gln Pro Pro Ala
    610                 615                 620

Asn Ala Pro Ser Asp Leu Thr Leu Gly Asn Glu Met Pro Lys Tyr Gly
625                 630                 635                 640

Tyr Gln Gly Ser Trp
                645

<210> 9
<211> 665
<212> PRT
<213> Chlamydia Trachomatis

<400> 9

Ser Pro Gln Val Leu Thr Pro Asn Val Thr Thr Pro Phe Lys Gly Asp
1               5                   10                  15

Asp Val Tyr Leu Asn Gly Asp Cys Ala Phe Val Asn Val Tyr Ala Gly
            20                  25                  30

Ala Glu Asn Gly Ser Ile Ile Ser Ala Asn Gly Asp Asn Leu Thr Ile
        35                  40                  45

Thr Gly Gln Asn His Thr Leu Ser Phe Thr His Ser Gln Gly Pro Val
    50                  55                  60

Leu Gln Asn Tyr Ala Phe Ile Ser Ala Gly Glu Thr Leu Thr Leu Lys
65                  70                  75                  80

Asp Phe Ser Ser Leu Met Phe Ser Lys Asn Val Ser Cys Gly Glu Lys
                85                  90                  95

Gly Met Ile Ser Gly Lys Thr Val Ser Ile Ser Gly Ala Gly Glu Val
            100                 105                 110

Ile Phe Arg Asp Asn Ser Val Gly Tyr Ser Pro Leu Ser Thr Val Pro
        115                 120                 125

Thr Ser Ser Ser Thr Pro Pro Ala Pro Asp Pro Ser Pro Thr Ala Ser
    130                 135                 140

Ser Ser Ser Ser Pro Thr Val Ser Asp Ala Arg Gln Gly Ser Ile Phe
145                 150                 155                 160

Ser Ile Glu Thr Ser Leu Glu Ile Ser Gly Ile Lys Lys Glu Val Met
                165                 170                 175

Phe Asp Asn Asn Ala Gly Asn Phe Gly Thr Val Phe Arg Gly Asn Ser
            180                 185                 190

Asn Asn Asn Ala Gly Ser Gly Gly Ser Gly Ser Ala Thr Thr Pro Ser
        195                 200                 205

Phe Thr Val Lys Asn Cys Lys Gly Lys Val Ser Phe Thr Asp Asn Val
    210                 215                 220

Ala Ser Cys Gly Gly Gly Val Val Tyr Lys Gly Thr Val Leu Phe Lys
225                 230                 235                 240

Asp Asn Glu Gly Gly Ile Phe Phe Arg Gly Asn Thr Ala Tyr Asp Asp

245                 250                 255

Leu Gly Ile Leu Ala Ala Thr Ser Gln Asp Gln Ser Asn Thr Glu Thr
            260                 265                 270

Gly Gly Gly Gly Gly Ala Ile Cys Ser Pro Glu Asp Thr Val Thr Phe
        275                 280                 285

Glu Gly Asn Lys Gly Ser Ile Val Phe Asp Tyr Asn Phe Ala Lys Gly
    290                 295                 300

Arg Gly Gly Gly Ile Leu Thr Lys Lys Phe Ser Leu Val Ala Asp Asp
305                 310                 315                 320

Ser Val Val Phe Ser Asn Asn Thr Ala Glu Lys Gly Gly Gly Ala Ile
                325                 330                 335

Tyr Ala Pro Thr Ile Asp Ile Ser Thr Asn Gly Gly Ser Ile Leu Phe
            340                 345                 350

Glu Arg Asn Arg Ala Ala Glu Gly Gly Ala Ile Cys Val Ser Glu Ala
        355                 360                 365

Ser Ser Gly Ser Thr Gly Asn Leu Thr Leu Ser Ala Ser Asp Gly Asp
    370                 375                 380

Ile Val Phe Ser Gly Asn Met Thr Ser Asp Arg Pro Gly Glu Arg Ser
385                 390                 395                 400

Ala Ala Arg Ile Leu Ser Asp Gly Thr Thr Val Ser Leu Asn Ala Ser
                405                 410                 415

Gly Leu Ser Lys Leu Ile Phe Tyr Asp Pro Val Val Gln Asn Asn Ser
            420                 425                 430

Ala Ala Gly Ala Ser Thr Pro Ser Pro Ser Ser Ser Ser Met Pro Gly
        435                 440                 445

Ala Val Thr Ile Asn Gln Ser Gly Asn Gly Ser Val Ile Phe Thr Ala
    450                 455                 460

Glu Ser Leu Thr Pro Ser Glu Lys Leu Gln Val Leu Asn Ser Thr Ser
465                 470                 475                 480

Asn Phe Pro Gly Ala Leu Thr Val Leu Gly Gly Glu Leu Val Val Thr
                485                 490                 495

Glu Gly Ala Thr Leu Thr Thr Gly Thr Ile Thr Ala Thr Ser Gly Arg
                500                 505                 510

Val Thr Leu Gly Ser Gly Ala Ser Leu Ser Ala Val Ala Gly Thr Ala
            515                 520                 525

Asn Asn Tyr Thr Cys Thr Val Ser Lys Leu Gly Val Asp Leu Glu Ser
    530                 535                 540

Phe Leu Thr Pro Asn Tyr Lys Thr Ala Ile Leu Gly Ala Asp Thr Thr
545                 550                 555                 560

Val Thr Val Asn Asn Asn Pro Thr Leu Asp Leu Val Met Lys Asn Glu
                565                 570                 575

Ala Glu Val Tyr Asp Asn Pro Leu Phe Val Gly Ser Leu Thr Ile Pro
                580                 585                 590

Phe Val Thr Leu Ser Ser Val Asn Asn Thr Asn Ser Asn Gly Val Val
            595                 600                 605

Lys Thr Ala Val Thr Ile Asn Asp Ala Asp Ala Ala His Tyr Gly Tyr
    610                 615                 620

Gln Gly Ser Trp Ser Ala Asp Trp Thr Lys Pro Pro Leu Ala Pro Asp
625                 630                 635                 640

Ala Lys Gly Met Val Pro Pro Asn Thr Ser Asn Thr Leu Tyr Leu Thr
                645                 650                 655

Trp Arg Pro Ala Ser Asn Tyr Gly Lys
                660                 665

<210>  10
<211>  433
<212>  PRT
<213>  Chlamydia Trachomatis

<400>  10

Met Leu Ile Phe Ala Leu Ser Cys Gly Ala Asp Ala Cys Leu Cys Ala
1               5                   10                  15

Ala Asp Leu Ser Lys Ala Lys Val Glu Ala Ser Val Gly Asp Arg Ala
            20                  25                  30

Ala Phe Ser Pro Phe Thr Gly Glu Ile Lys Gly Asn Arg Val Arg Leu
        35                  40                  45

Arg Leu Ala Pro His Thr Asp Ser Phe Ile Ile Lys Glu Leu Ser Lys
    50                  55                  60

Gly Asp Cys Leu Ala Val Leu Gly Glu Ser Lys Asp Tyr Tyr Val Val
65                  70                  75                  80

Ala Ala Pro Glu Gly Val Arg Gly Tyr Val Phe Arg Thr Phe Val Leu
                85                  90                  95

Asp Asn Val Ile Glu Gly Glu Lys Val Asn Val Arg Leu Glu Pro Ser
            100                 105                 110

Thr Ser Ala Pro Ile Leu Ala Arg Leu Ser Lys Gly Thr Val Val Lys
        115                 120                 125

Thr Leu Gly Ala Ala Gln Gly Lys Trp Ile Glu Ile Ala Leu Pro Lys
    130                 135                 140

Gln Cys Val Phe Tyr Val Ala Lys Asn Phe Val Lys Asn Val Gly Ala
145                 150                 155                 160

Leu Asp Leu Tyr Asn Gln Lys Glu Gly Gln Lys Lys Leu Ala Leu Asp
                165                 170                 175

Leu Leu Ser Ser Ala Met Asn Phe Ala Asp Ala Glu Leu Gln Lys Lys
            180                 185                 190

Ile Glu Asp Ile Asp Leu Asp Ala Ile Tyr Lys Lys Met Asn Leu Ala
        195                 200                 205

Gln Ser Glu Glu Phe Lys Asp Val Pro Gly Leu Gln Ser Leu Val Gln
    210                 215                 220

Lys Ala Leu Glu Arg Val Gln Glu Ala Phe Leu Ala Lys Ser Leu Glu
225                 230                 235                 240

Lys Ser Ser Val Lys Val Pro Glu Ile Gln His Glu Val Leu Glu Glu
                245                 250                 255

Ile Ala Val Val Ser Pro Ala Val Glu Glu Thr Pro Val Val Thr Lys
            260                 265                 270

Ile Glu Glu Gln Lys Val Thr Thr Val Pro Val Pro Ala Pro Ala Val
        275                 280                 285

Val Thr Glu Pro Ala Gln Asp Leu Ser Ser Val Lys Gly Ser Leu Leu
    290                 295                 300

Ser His Tyr Ile Arg Lys Lys Gly Phe Val Lys Ala Ser Pro Val Ile
305                 310                 315                 320

Glu Gly Arg Glu Ser Phe Glu Arg Ser Leu Phe Ala Val Trp Val Ser
                325                 330                 335

Leu Gln Pro Glu Glu Ile Gln His Gln Leu Thr Met Glu Ser Phe Tyr
            340                 345                 350

Arg Asp Glu Gln Lys Lys Lys Arg Val Leu Thr Gly Glu Leu Glu Val
        355                 360                 365

Tyr Pro His Ile Val Lys Asn Asn Pro Gly Asp Tyr Leu Leu Lys Asn
    370                 375                 380

Gly Glu Asp Val Val Ala Phe Val Tyr Ala Thr Ser Ile Asp Leu Ser
385                 390                 395                 400

Lys Trp Leu Gly Lys Ser Val Val Leu Glu Cys Val Ser Arg Pro Asn
                405                 410                 415

Asn His Phe Ala Phe Pro Ala Tyr Ile Val Leu Ser Val Lys Glu Gly
            420                 425                 430

Ala

<210>  11
<211>  612
<212>  PRT
<213>  Chlamydia Trachomatis

<400>  11

Glu Leu Ala Ile Ser Gly His Lys Gln Gly Lys Asp Arg Asp Thr Phe
1               5                   10                  15

Thr Met Ile Ser Ser Cys Pro Glu Gly Thr Asn Tyr Ile Ile Asn Arg
            20                  25                  30

Lys Leu Ile Leu Ser Asp Phe Ser Leu Leu Asn Lys Val Ser Ser Gly
        35                  40                  45

Gly Ala Phe Arg Asn Leu Ala Gly Lys Ile Ser Phe Leu Gly Lys Asn
    50                  55                  60

Ser Ser Ala Ser Ile His Phe Lys His Ile Asn Ile Asn Gly Phe Gly
65                  70                  75                  80

Ala Gly Val Phe Ser Glu Ser Ser Ile Glu Phe Thr Asp Leu Arg Lys
                85                  90                  95

Leu Val Ala Phe Gly Ser Glu Ser Thr Gly Gly Ile Phe Thr Ala Lys
            100                 105                 110

Glu Asp Ile Ser Phe Lys Asn Asn His His Ile Ala Phe Arg Asn Asn
        115                 120                 125

Ile Thr Lys Gly Asn Gly Gly Val Ile Gln Leu Gln Gly Asp Met Lys
    130                 135                 140

Gly Ser Val Ser Phe Val Asp Gln Arg Gly Ala Ile Ile Phe Thr Asn
145                 150                 155                 160

Asn Gln Ala Val Thr Ser Ser Ser Met Lys His Ser Gly Arg Gly Gly
                165                 170                 175

Ala Ile Ser Gly Asp Phe Ala Gly Ser Arg Ile Leu Phe Leu Asn Asn
            180                 185                 190

Gln Gln Ile Thr Phe Glu Gly Asn Ser Ala Val His Gly Gly Ala Ile
        195                 200                 205

Tyr Asn Lys Asn Gly Leu Val Glu Phe Leu Gly Asn Ala Gly Pro Leu
    210                 215                 220

Ala Phe Lys Glu Asn Thr Thr Ile Ala Asn Gly Gly Ala Ile Tyr Thr
225                 230                 235                 240

Ser Asn Phe Lys Ala Asn Gln Gln Thr Ser Pro Ile Leu Phe Ser Gln
                245                 250                 255

Asn His Ala Asn Lys Lys Gly Gly Ala Ile Tyr Ala Gln Tyr Val Asn
            260                 265                 270

Leu Glu Gln Asn Gln Asp Thr Ile Arg Phe Glu Lys Asn Thr Ala Lys
        275                 280                 285

Glu Gly Gly Gly Ala Ile Thr Ser Ser Gln Cys Ser Ile Thr Ala His
    290                 295                 300

Asn Thr Ile Thr Phe Ser Asp Asn Ala Ala Gly Asp Leu Gly Gly Gly
305                 310                 315                 320

Ala Ile Leu Leu Glu Gly Lys Lys Pro Ser Leu Thr Leu Ile Ala His
                325                 330                 335

Ser Gly Asn Ile Ala Phe Ser Gly Asn Thr Met Leu His Ile Thr Lys
            340                 345                 350

Lys Ala Ser Leu Asp Arg His Asn Ser Ile Leu Ile Lys Glu Ala Pro
        355                 360                 365

Tyr Lys Ile Gln Leu Ala Ala Asn Lys Asn His Ser Ile His Phe Phe
    370                 375                 380

Asp Pro Val Met Ala Leu Ser Ala Ser Ser Ser Pro Ile Gln Ile Asn
385                 390                 395                 400

Ala Pro Glu Tyr Glu Thr Pro Phe Phe Ser Pro Lys Gly Met Ile Val
                405                 410                 415

Phe Ser Gly Ala Asn Leu Leu Asp Asp Ala Arg Glu Asp Val Ala Asn
            420                 425                 430

Arg Thr Ser Ile Phe Asn Gln Pro Val His Leu Tyr Asn Gly Thr Leu
        435                 440                 445

Ser Ile Glu Asn Gly Ala His Leu Ile Val Gln Ser Phe Lys Gln Thr
    450                 455                 460

Gly Gly Arg Ile Ser Leu Ser Pro Gly Ser Ser Leu Ala Leu Tyr Thr
465                 470                 475                 480

Met Asn Ser Phe Phe His Gly Asn Ile Ser Ser Lys Glu Pro Leu Glu
                485                 490                 495

Ile Asn Gly Leu Ser Phe Gly Val Asp Ile Ser Pro Ser Asn Leu Gln
            500                 505                 510

Ala Glu Ile Arg Ala Gly Asn Ala Pro Leu Arg Leu Ser Gly Ser Pro
        515                 520                 525

Ser Ile His Asp Pro Glu Gly Leu Phe Tyr Glu Asn Arg Asp Thr Ala
    530                 535                 540

Ala Ser Pro Tyr Gln Met Glu Ile Leu Leu Thr Ser Asp Lys Thr Val
545                 550                 555                 560

Asp Ile Ser Lys Phe Thr Thr Asp Ser Leu Val Thr Asn Lys Gln Ser
                565                 570                 575

Gly Phe Gln Gly Ala Trp His Phe Ser Trp Gln Pro Asn Thr Ile Asn

            580                 585                 590

Asn Thr Lys Gln Lys Ile Leu Arg Ala Ser Trp Leu Pro Thr Gly Glu
        595                 600                 605

Tyr Val Leu Glu
    610

<210> 12
<211> 585
<212> PRT
<213> Chlamydia Trachomatis

<400> 12

Leu His Ala Gly Gly Gln Ser Val Asn Glu Leu Val Tyr Val Gly Pro
1               5                   10                  15

Gln Ala Val Leu Leu Leu Asp Gln Ile Arg Asp Leu Phe Val Gly Ser
            20                  25                  30

Lys Asp Ser Gln Ala Glu Gly Gln Tyr Arg Leu Ile Val Gly Asp Pro
        35                  40                  45

Ser Ser Phe Gln Glu Lys Asp Ala Asp Thr Leu Pro Gly Lys Val Glu
    50                  55                  60

Gln Ser Thr Leu Phe Ser Val Thr Asn Pro Val Val Phe Gln Gly Val
65                  70                  75                  80

Asp Gln Gln Asp Gln Val Ser Ser Gln Gly Leu Ile Cys Ser Phe Thr
                85                  90                  95

Ser Ser Asn Leu Asp Ser Pro Arg Asp Gly Glu Ser Phe Leu Gly Ile
            100                 105                 110

Ala Phe Val Gly Asp Ser Ser Lys Ala Gly Ile Thr Leu Thr Asp Val
        115                 120                 125

Lys Ala Ser Leu Ser Gly Ala Ala Leu Tyr Ser Thr Glu Asp Leu Ile
    130                 135                 140

Phe Glu Lys Ile Lys Gly Gly Leu Glu Phe Ala Ser Cys Ser Ser Leu
145                 150                 155                 160

Glu Gln Gly Gly Ala Cys Ala Ala Gln Ser Ile Leu Ile His Asp Cys
                165                 170                 175

Gln Gly Leu Gln Val Lys His Cys Thr Thr Ala Val Asn Ala Glu Gly

            180                 185                 190

Ser Ser Ala Asn Asp His Leu Gly Phe Gly Gly Gly Ala Phe Phe Val
        195                 200                 205

Thr Gly Ser Leu Ser Gly Glu Lys Ser Leu Tyr Met Pro Ala Gly Asp
    210                 215                 220

Met Val Val Ala Asn Cys Asp Gly Ala Ile Ser Phe Glu Gly Asn Ser
225                 230                 235                 240

Ala Asn Phe Ala Asn Gly Gly Ala Ile Ala Ala Ser Gly Lys Val Leu
                245                 250                 255

Phe Val Ala Asn Asp Lys Lys Thr Ser Phe Ile Glu Asn Arg Ala Leu
            260                 265                 270

Ser Gly Gly Ala Ile Ala Ala Ser Ser Asp Ile Ala Phe Gln Asn Cys
        275                 280                 285

Ala Glu Leu Val Phe Lys Gly Asn Cys Ala Ile Gly Thr Glu Asp Lys
    290                 295                 300

Gly Ser Leu Gly Gly Gly Ala Ile Ser Ser Leu Gly Thr Val Leu Leu
305                 310                 315                 320

Gln Gly Asn His Gly Ile Thr Cys Asp Lys Asn Glu Ser Ala Ser Gln
                325                 330                 335

Gly Gly Ala Ile Phe Gly Lys Asn Cys Gln Ile Ser Asp Asn Glu Gly
            340                 345                 350

Pro Val Val Phe Arg Asp Ser Thr Ala Cys Leu Gly Gly Gly Ala Ile
        355                 360                 365

Ala Ala Gln Glu Ile Val Ser Ile Gln Asn Asn Gln Ala Gly Ile Ser
    370                 375                 380

Phe Glu Gly Gly Lys Ala Ser Phe Gly Gly Gly Ile Ala Cys Gly Ser
385                 390                 395                 400

Phe Ser Ser Ala Gly Gly Ala Ser Val Leu Gly Thr Ile Asp Ile Ser
                405                 410                 415

Lys Asn Leu Gly Ala Ile Ser Phe Ser Arg Thr Leu Cys Thr Thr Ser
            420                 425                 430

Asp Leu Gly Gln Met Glu Tyr Gln Gly Gly Gly Ala Leu Phe Gly Glu
        435                 440                 445

Asn Ile Ser Leu Ser Glu Asn Ala Gly Val Leu Thr Phe Lys Asp Asn
    450                 455                 460

Ile Val Lys Thr Phe Ala Ser Asn Gly Lys Ile Leu Gly Gly Gly Ala
465                 470                 475                 480

Ile Leu Ala Thr Gly Lys Val Glu Ile Thr Asn Asn Ser Gly Gly Ile
                485                 490                 495

Ser Phe Thr Gly Asn Ala Arg Ala Pro Gln Ala Leu Pro Thr Gln Glu
            500                 505                 510

Glu Phe Pro Leu Phe Ser Lys Lys Glu Gly Arg Pro Leu Ser Ser Gly
        515                 520                 525

Tyr Ser Gly Gly Gly Ala Ile Leu Gly Arg Glu Val Ala Ile Leu His
    530                 535                 540

Asn Ala Ala Val Val Phe Glu Gln Asn Arg Leu Gln Cys Ser Glu Glu
545                 550                 555                 560

Glu Ala Thr Leu Leu Gly Cys Cys Gly Gly Gly Ala Val His Gly Met
                565                 570                 575

Asp Ser Thr Ser Ile Val Gly Asn Ser
            580                 585

<210>  13
<211>  645
<212>  PRT
<213>  Chlamydia Trachomatis

<400>  13

Glu Ile Met Val Pro Gln Gly Ile Tyr Asp Gly Glu Thr Leu Thr Val
1               5                   10                  15

Ser Phe Pro Tyr Thr Val Ile Gly Asp Pro Ser Gly Thr Thr Val Phe
            20                  25                  30

Ser Ala Gly Glu Leu Thr Leu Lys Asn Leu Asp Asn Ser Ile Ala Ala
        35                  40                  45

Leu Pro Leu Ser Cys Phe Gly Asn Leu Leu Gly Ser Phe Thr Val Leu
    50                  55                  60

Gly Arg Gly His Ser Leu Thr Phe Glu Asn Ile Arg Thr Ser Thr Asn
65                  70                  75                  80

Gly Ala Ala Leu Ser Asn Ser Ala Ala Asp Gly Leu Phe Thr Ile Glu
                85                  90                  95

Gly Phe Lys Glu Leu Ser Phe Ser Asn Cys Asn Ser Leu Leu Ala Val
            100                 105                 110

Leu Pro Ala Ala Thr Thr Asn Lys Gly Ser Gln Thr Pro Thr Thr Thr
        115                 120                 125

Ser Thr Pro Ser Asn Gly Thr Ile Tyr Ser Lys Thr Asp Leu Leu Leu
    130                 135                 140

Leu Asn Asn Glu Lys Phe Ser Phe Tyr Ser Asn Leu Val Ser Gly Asp
145                 150                 155                 160

Gly Gly Ala Ile Asp Ala Lys Ser Leu Thr Val Gln Gly Ile Ser Lys
                165                 170                 175

Leu Cys Val Phe Gln Glu Asn Thr Ala Gln Ala Asp Gly Gly Ala Cys
            180                 185                 190

Gln Val Val Thr Ser Phe Ser Ala Met Ala Asn Glu Ala Pro Ile Ala
        195                 200                 205

Phe Val Ala Asn Val Ala Gly Val Arg Gly Gly Gly Ile Ala Ala Val
    210                 215                 220

Gln Asp Gly Gln Gln Gly Val Ser Ser Ser Thr Ser Thr Glu Asp Pro
225                 230                 235                 240

Val Val Ser Phe Ser Arg Asn Thr Ala Val Glu Phe Asp Gly Asn Val
                245                 250                 255

Ala Arg Val Gly Gly Gly Ile Tyr Ser Tyr Gly Asn Val Ala Phe Leu
            260                 265                 270

Asn Asn Gly Lys Thr Leu Phe Leu Asn Asn Val Ala Ser Pro Val Tyr
        275                 280                 285

Ile Ala Ala Lys Gln Pro Thr Ser Gly Gln Ala Ser Asn Thr Ser Asn
    290                 295                 300

Asn Tyr Gly Asp Gly Gly Ala Ile Phe Cys Lys Asn Gly Ala Gln Ala
305                 310                 315                 320

Gly Ser Asn Asn Ser Gly Ser Val Ser Phe Asp Gly Glu Gly Val Val
                325                 330                 335

Phe Phe Ser Ser Asn Val Ala Ala Gly Lys Gly Gly Ala Ile Tyr Ala
            340                 345                 350

Lys Lys Leu Ser Val Ala Asn Cys Gly Pro Val Gln Phe Leu Arg Asn
        355                 360                 365

Ile Ala Asn Asp Gly Gly Ala Ile Tyr Leu Gly Glu Ser Gly Glu Leu
    370                 375                 380

Ser Leu Ser Ala Asp Tyr Gly Asp Ile Ile Phe Asp Gly Asn Leu Lys
385                 390                 395                 400

Arg Thr Ala Lys Glu Asn Ala Ala Asp Val Asn Gly Val Thr Val Ser
                405                 410                 415

Ser Gln Ala Ile Ser Met Gly Ser Gly Gly Lys Ile Thr Thr Leu Arg
            420                 425                 430

Ala Lys Ala Gly His Gln Ile Leu Phe Asn Asp Pro Ile Glu Met Ala
        435                 440                 445

Asn Gly Asn Asn Gln Pro Ala Gln Ser Ser Lys Leu Leu Lys Ile Asn
    450                 455                 460

Asp Gly Glu Gly Tyr Thr Gly Asp Ile Val Phe Ala Asn Gly Ser Ser
465                 470                 475                 480

Thr Leu Tyr Gln Asn Val Thr Ile Glu Gln Gly Arg Ile Val Leu Arg
                485                 490                 495

Glu Lys Ala Lys Leu Ser Val Asn Ser Leu Ser Gln Thr Gly Gly Ser
            500                 505                 510

Leu Tyr Met Glu Ala Gly Ser Thr Leu Asp Phe Val Thr Pro Gln Pro
        515                 520                 525

Pro Gln Gln Pro Pro Ala Ala Asn Gln Leu Ile Thr Leu Ser Asn Leu
    530                 535                 540

His Leu Ser Leu Ser Ser Leu Leu Ala Asn Asn Ala Val Thr Asn Pro
545                 550                 555                 560

Pro Thr Asn Pro Pro Ala Gln Asp Ser His Pro Ala Val Ile Gly Ser
                565                 570                 575

Thr Thr Ala Gly Ser Val Thr Ile Ser Gly Pro Ile Phe Phe Glu Asp
            580                 585                 590

Leu Asp Asp Thr Ala Tyr Asp Arg Tyr Asp Trp Leu Gly Ser Asn Gln
        595                 600                 605

Lys Ile Asn Val Leu Lys Leu Gln Leu Gly Thr Lys Pro Pro Ala Asn
    610                 615                 620

Ala Pro Ser Asp Leu Thr Leu Gly Asn Glu Met Pro Lys Tyr Gly Tyr
625                 630                 635                 640

Gln Gly Ser Trp Lys
                645

<210>  14
<211>  665
<212>  PRT
<213>  Chlamydia Trachomatis

<400>  14

Ser Pro Gln Val Leu Thr Pro Asn Val Thr Thr Pro Phe Lys Gly Asp
1               5                   10                  15

Asp Val Tyr Leu Asn Gly Asp Cys Ala Phe Val Asn Val Tyr Ala Gly
            20                  25                  30

Ala Glu Glu Gly Ser Ile Ile Ser Ala Asn Gly Asp Asn Leu Thr Ile
        35                  40                  45

Thr Gly Gln Asn His Thr Leu Ser Phe Thr Asp Ser Gln Gly Pro Val
    50                  55                  60

Leu Gln Asn Tyr Ala Phe Ile Ser Ala Gly Glu Thr Leu Thr Leu Arg
65                  70                  75                  80

Asp Phe Ser Ser Leu Met Phe Ser Lys Asn Val Ser Cys Gly Glu Lys
                85                  90                  95

Gly Met Ile Ser Gly Lys Thr Val Ser Ile Ser Gly Ala Gly Glu Val
            100                 105                 110

Ile Phe Trp Asp Asn Ser Val Gly Tyr Ser Pro Leu Ser Thr Val Pro
        115                 120                 125

Thr Ser Ser Ser Thr Pro Pro Ala Pro Thr Val Ser Asp Ala Arg Lys
    130                 135                 140

Gly Ser Ile Phe Ser Val Glu Thr Ser Leu Glu Ile Ser Gly Val Lys
145                 150                 155                 160

Lys Gly Val Met Phe Asp Asn Asn Ala Gly Asn Phe Gly Thr Val Phe
                165                 170                 175

Arg Gly Lys Asn Asn Asn Asn Ala Gly Gly Gly Gly Ser Gly Ser Ala
            180                 185                 190

Thr Pro Ser Ser Thr Thr Phe Thr Val Lys Asn Cys Lys Gly Lys Val
        195                 200                 205

Ser Phe Thr Asp Asn Val Ala Ser Cys Gly Gly Gly Val Val Tyr Lys
    210                 215                 220

Gly Ile Val Leu Phe Lys Asp Asn Glu Gly Gly Ile Phe Phe Arg Gly
225                 230                 235                 240

Asn Thr Ala Tyr Asp Asp Leu Arg Ile Leu Ala Ala Thr Asn Gln Asp
                245                 250                 255

Gln Asn Thr Glu Thr Gly Gly Gly Gly Gly Val Ile Cys Ser Pro Asp
            260                 265                 270

Asp Ser Val Lys Phe Glu Gly Asn Lys Gly Ser Ile Val Phe Asp Tyr
        275                 280                 285

Asn Phe Ala Lys Gly Arg Gly Gly Ser Ile Leu Thr Lys Glu Phe Ser
    290                 295                 300

Leu Val Ala Asp Asp Ser Val Val Phe Ser Asn Asn Thr Ala Glu Lys
305                 310                 315                 320

Gly Gly Gly Ala Ile Tyr Ala Pro Thr Ile Asn Met Ser Thr Asn Gly
                325                 330                 335

Gly Ser Ile Leu Phe Glu Arg Asn Arg Ala Ala Glu Gly Gly Ala Ile
            340                 345                 350

Cys Val Ser Ala Ala Ser Ser Gly Ser Thr Gly Lys Leu Thr Leu Ser
        355                 360                 365

Ala Ser Asp Gly Asp Ile Val Phe Ser Gly Asn Met Thr Ser Asp Arg
    370                 375                 380

Pro Gly Glu Arg Ser Ala Val Arg Ile Leu Ser Ser Gly Thr Thr Val

385                 390                 395                 400

Ala Leu Asn Ala Ser Gly Leu Ser Lys Leu Ile Phe His Asp Pro Val
                405                 410                 415

Val Gln Asn Asn Ser Ala Ala Ala Ala Ser Thr Pro Ser Pro Ser Ser
            420                 425                 430

Ser Ser Thr Pro Ala Ala Val Thr Ile Asn Gln Ser Gly Asn Gly Ser
        435                 440                 445

Val Ile Phe Thr Ala Glu Ser Leu Thr Pro Ser Glu Lys Leu Gln Val
    450                 455                 460

Leu Asn Ser Thr Ser Asn Phe Pro Gly Thr Leu Thr Val Ser Gly Gly
465                 470                 475                 480

Glu Leu Val Val Thr Glu Gly Ala Thr Leu Thr Thr Gly Thr Ile Thr
                485                 490                 495

Ala Thr Ser Gly Arg Val Thr Leu Gly Ser Gly Ala Ser Leu Ser Ala
            500                 505                 510

Val Ala Gly Ala Ala Asn Asn Asn Tyr Thr Cys Thr Val Ser Lys Leu
        515                 520                 525

Gly Ile Asp Leu Glu Ser Phe Leu Thr Pro Asn Tyr Lys Thr Ala Ile
    530                 535                 540

Leu Gly Ala Tyr Gly Thr Val Thr Val Lys Arg Gly Ser Thr Leu Asp
545                 550                 555                 560

Leu Val Met Glu Asn Glu Ala Glu Val Tyr Asp Asn Pro Leu Phe Val
                565                 570                 575

Gly Ser Leu Thr Ile Pro Phe Val Thr Leu Ser Ser Ser Ser Thr Ser
            580                 585                 590

Asn Gly Val Thr Met Lys Arg Val Thr Ile Asn Asp Ala Asp Ala Ala
        595                 600                 605

His Tyr Gly Tyr Gln Gly Ser Trp Ser Ala Asp Trp Thr Lys Pro Pro
    610                 615                 620

Leu Ala Pro Asp Ala Lys Gly Met Val Pro Pro Asn Thr Ser Asn Thr
625                 630                 635                 640

Leu Tyr Leu Thr Trp Arg Pro Ala Ser Asn Tyr Gly Glu Tyr Arg Leu
                645                 650                 655

Asp Pro Gln Arg Lys Gly Glu Leu Val
            660                 665

<210> 15
<211> 433
<212> PRT
<213> Chlamydia Trachomatis

<400> 15

Met Thr Phe Ser Phe Lys Ser Ile Ile Ile Pro Ser Leu Glu Asp Glu
1               5                   10                  15

Ser Ser Arg Ala Val Asn Ala Ser Leu Glu Ala Gln Ile Leu Ala Asn
            20                  25                  30

Cys Arg Ser Phe Cys Val Arg Thr Leu Ser Ile Ser Met Leu Ile Phe
        35                  40                  45

Ala Leu Ser Cys Gly Ala Asp Ala Cys Leu Cys Ala Ala Asp Leu Ser
    50                  55                  60

Lys Ala Lys Val Glu Ala Ser Val Gly Asp Arg Ala Ala Phe Ser Pro
65                  70                  75                  80

Phe Thr Gly Glu Ile Lys Gly Asn Arg Val Arg Leu Arg Leu Ala Pro
                85                  90                  95

His Thr Asp Ser Phe Ile Ile Lys Glu Leu Ser Lys Gly Asp Cys Leu
            100                 105                 110

Ala Val Leu Gly Glu Ser Lys Asp Tyr Tyr Val Val Ala Ala Pro Glu
        115                 120                 125

Gly Val Arg Gly Tyr Val Phe Arg Thr Phe Val Leu Asp Asn Val Ile
    130                 135                 140

Glu Gly Glu Lys Val Asn Val Arg Leu Glu Pro Ser Thr Ser Ala Pro
145                 150                 155                 160

Ile Leu Ala Arg Leu Ser Lys Gly Thr Val Val Lys Thr Leu Gly Ala
                165                 170                 175

Ala Gln Gly Lys Trp Ile Glu Ile Ala Leu Pro Lys Gln Cys Val Phe
            180                 185                 190

Tyr Val Ala Lys Asn Phe Val Lys Asn Val Gly Ala Leu Asp Leu Tyr
        195                 200                 205

Asn Gln Lys Glu Gly Gln Lys Lys Leu Ala Leu Asp Leu Leu Ser Ser
    210                 215                 220

Ala Met Asp Phe Ala Asp Ala Glu Leu Gln Lys Lys Ile Glu Asp Ile
225                 230                 235                 240

Asp Leu Asp Ala Ile Tyr Lys Lys Met Asn Leu Ala Gln Ser Glu Glu
                245                 250                 255

Phe Lys Asp Val Pro Gly Leu Gln Ser Leu Val Gln Lys Ala Leu Glu
            260                 265                 270

Arg Val Gln Glu Ala Phe Leu Ala Lys Ser Leu Glu Lys Ser Ala Val
        275                 280                 285

Lys Val Pro Glu Ile Gln His Lys Val Leu Glu Glu Ile Ala Val Val
    290                 295                 300

Ser Pro Ala Val Glu Glu Thr Pro Val Val Thr Lys Ile Glu Glu Gln
305                 310                 315                 320

Lys Val Thr Thr Val Pro Val Pro Ala Pro Ala Val Val Thr Glu Leu
                325                 330                 335

Ala Gln Asp Leu Ser Ser Val Lys Gly Ser Leu Leu Ser His Tyr Ile
            340                 345                 350

Arg Lys Lys Gly Phe Val Lys Ala Ser Pro Val Ile Glu Gly Arg Glu
        355                 360                 365

Ser Phe Glu Arg Ser Leu Phe Ala Val Trp Val Ser Leu Gln Pro Glu
    370                 375                 380

Glu Ile Arg His Gln Leu Thr Met Glu Ser Phe Tyr Arg Asp Glu Gln
385                 390                 395                 400

Lys Lys Lys Arg Val Leu Thr Gly Glu Leu Glu Val Tyr Pro His Ile
                405                 410                 415

Val Lys Asn Asn Pro Gly Asp Tyr Leu Leu Lys Asn Gly Glu Asp Val
            420                 425                 430

Val

<210>  16
<211>  613
<212>  PRT
<213>  C. muridarum

<400>  16

Glu Met Glu Leu Ala Ile Ser Gly His Lys Tyr Gly Lys Asp Arg Asp
1               5                   10                  15

Ala Phe Thr Met Ile Ser Ser Cys Pro Glu Gly Thr Asn Tyr Met Ile
            20                  25                  30

Asn Arg Lys Leu Ile Leu Ser Glu Phe Ser Ser Leu Asp Lys Phe Ser
        35                  40                  45

Ser Gly Gly Ala Phe Lys Asn Leu Ala Gly Lys Ile Ala Phe Leu Gly
    50                  55                  60

Lys His Ser Ser Ser Ser Ile His Phe Lys His Leu Asn Ile Asn Gly
65                  70                  75                  80

Phe Gly Ser Gly Ile Phe Ser Glu Ser Ala Ile Glu Phe Ser Asp Leu
                85                  90                  95

Arg Lys Leu Val Ala Phe Gly Ser Glu Ser Thr Gly Gly Ile Phe Thr
            100                 105                 110

Ala Arg Asp Asp Ile Ser Phe Lys Asn Asn His Tyr Ile Ala Phe Arg
        115                 120                 125

Asn Asn Ile Ala Lys Gly Asn Gly Gly Val Ile Leu Leu Gln Gly Asp
    130                 135                 140

Glu Arg Gly Thr Val Ser Phe Thr Asp Gln Gln Gly Ala Ile Ile Phe
145                 150                 155                 160

Ala Asn Asn Gln Ala Leu Val Ser Pro Ser Ile Lys His Ser Gly Arg
                165                 170                 175

Gly Gly Ala Ile Ser Gly Asp Phe Ala Gly Ser Arg Ile Ile Phe Leu
            180                 185                 190

Asn Asn Gln Gln Ile Thr Phe Glu Glu Asn Ser Ala Val His Gly Gly
        195                 200                 205

Ala Ile Tyr Asn Lys Asn Gly Val Val Glu Phe Leu Gly Asn Gly Gly
    210                 215                 220

Thr Leu Ser Phe Lys Glu Asn Ser Thr Arg Ala Asn Gly Gly Ala Ile
225                 230                 235                 240

Tyr Thr Gly Lys Phe Lys Ala Asn Gln Gln Thr Ala Pro Ile Ile Phe
                245                 250                 255

Ser Gln Asn Asn Ala Asn Gln Lys Gly Gly Ala Ile Tyr Ala Gln Tyr
            260                 265                 270

Val Asn Leu Glu Gln Asn Gln Asp Ala Ile Arg Phe Glu Asn Asn Ser
        275                 280                 285

Ala Lys Glu Gly Gly Gly Ala Ile Ser Ser Ser Gln Cys Ala Ile Thr
    290                 295                 300

Ala His Asn Ser Ile Thr Phe Ser Asn Asn Phe Ala Gly Asp Leu Gly
305                 310                 315                 320

Gly Gly Ala Ile Leu Leu Gly Gly Lys Gln Pro Ser Leu Ser Leu Val
                325                 330                 335

Ala His Asn Gly Asn Ile Ala Phe Ile Gly Asn Thr Met Leu Pro Ala
            340                 345                 350

Thr Lys Lys Ala Ser Leu Pro Arg Asn Asn Ser Ile Leu Val Lys Glu
        355                 360                 365

Ser Pro Tyr Lys Ile Gln Phe Ala Ala Asn Lys Asn Arg Ser Ile Ile
    370                 375                 380

Phe Phe Asp Pro Val Ile Ala Leu Ser Pro Ser Thr Ser Pro Val Glu
385                 390                 395                 400

Ile Asn Ser Pro Glu His Glu Thr Pro Phe Phe Ser Pro Lys Gly Thr
                405                 410                 415

Ile Val Phe Ser Gly Ala Asn Leu Ile Asp Asp Ala Lys Glu Asp Ile
            420                 425                 430

Ala Asn Arg Thr Ser Ile Phe Asn Gln Pro Val Leu Leu His Asn Gly
        435                 440                 445

Thr Leu Ser Ile Glu Ser Gly Ala His Leu Val Val Gln Ser Phe Lys
    450                 455                 460

Gln Thr Gly Gly Arg Ile Ser Leu Ser Pro Gly Ser Ser Leu Ala Leu
465                 470                 475                 480

Tyr Thr Thr Asn Thr Leu Phe His Gly Asn Val Ser Ser Thr Asp Pro
                485                 490                 495

Leu Glu Ile Asn Gly Leu Ser Leu Gly Val Asp Thr Ser Pro Ser Asn
            500                 505                 510

Leu Tyr Ser Glu Ile Arg Ala Gly Ser Ala Pro Leu Lys Leu Ser Gly
        515                 520                 525

Ser Pro Asp Ile His Asp Pro Glu Arg Leu Phe Tyr Glu Asn Arg Asp
    530                 535                 540

Ser Ala Ala Ser Pro Tyr Gln Met Glu Ile Leu Leu Ser Ser Asp Lys
545                 550                 555                 560

Ile Ile Asp Val Ser Asn Phe Thr Ile Asp Ala Pro Val Pro Asn Lys
                565                 570                 575

Glu Ala Gly Phe Gln Gly Ser Trp His Phe Ser Trp Gln Pro Asn Thr
            580                 585                 590

Val Asn Asn Thr Lys His Lys Val Leu Arg Ala Ser Trp Ile Pro Thr
        595                 600                 605

Gly Glu Tyr Ile Leu
    610

<210>  17
<211>  586
<212>  PRT
<213>  C. muridarum

<400>  17

Asp Leu Tyr Ala Val Gly Ser Ser Ala Asp His Pro Ala Tyr Leu Ile
1               5                   10                  15

Pro Gln Ala Gly Leu Leu Leu Asp His Ile Lys Asp Ile Phe Ile Gly
            20                  25                  30

Pro Lys Asp Ser Gln Asp Lys Gly Gln Tyr Lys Leu Ile Ile Gly Glu
        35                  40                  45

Ala Gly Ser Phe Gln Asp Ser Asn Ala Glu Thr Leu Pro Gln Lys Val
    50                  55                  60

Glu His Ser Thr Leu Phe Ser Val Thr Thr Pro Ile Ile Val Gln Gly
65                  70                  75                  80

Ile Asp Gln Gln Asp Gln Val Ser Ser Gln Gly Leu Val Cys Asn Phe
                85                  90                  95

Ser Gly Asp His Ser Glu Glu Ile Phe Glu Arg Glu Ser Phe Leu Gly
            100                 105                 110

Ile Ala Phe Leu Gly Asn Gly Ser Lys Asp Gly Ile Thr Leu Thr Asp
        115                 120                 125

Ile Lys Ser Ser Leu Ser Gly Ala Ala Leu Tyr Ser Ser Asp Asp Leu
    130                 135                 140

Ile Phe Glu Arg Ile Lys Gly Asp Ile Glu Leu Ser Ser Cys Ser Ser
145                 150                 155                 160

Leu Glu Arg Gly Gly Ala Cys Ser Ala Gln Ser Ile Leu Ile His Asp
                165                 170                 175

Cys Gln Gly Leu Thr Val Lys His Cys Ala Ala Gly Val Asn Val Glu
            180                 185                 190

Gly Val Ser Ala Ser Asp His Leu Gly Phe Gly Gly Gly Ala Phe Ser
        195                 200                 205

Thr Thr Ser Ser Leu Ser Gly Glu Lys Ser Leu Tyr Met Pro Ala Gly
    210                 215                 220

Asp Ile Val Val Ala Thr Cys Asp Gly Pro Val Cys Phe Glu Gly Asn
225                 230                 235                 240

Ser Ala Gln Leu Ala Asn Gly Gly Ala Ile Ala Ala Ser Gly Lys Val
                245                 250                 255

Leu Phe Val Ala Asn Glu Lys Lys Ile Ser Phe Thr Asp Asn Gln Ala
            260                 265                 270

Leu Ser Gly Gly Ala Ile Ser Ala Ser Ser Ser Ile Ser Phe Gln Asn
        275                 280                 285

Cys Ala Glu Leu Val Phe Lys Ser Asn Leu Ala Lys Gly Val Lys Asp
    290                 295                 300

Lys Cys Ser Leu Gly Gly Gly Ala Leu Ala Ser Leu Glu Ser Val Val
305                 310                 315                 320

Leu Lys Asp Asn Leu Gly Ile Thr Tyr Glu Lys Asn Gln Ser Tyr Ser

325                 330                 335

Glu Gly Gly Ala Ile Phe Gly Lys Asp Cys Glu Ile Phe Glu Asn Arg
        340                 345                 350

Gly Pro Val Val Phe Arg Asp Asn Thr Ala Ala Leu Gly Gly Gly Ala
        355                 360                 365

Ile Leu Ala Gln Gln Thr Val Ala Ile Cys Gly Asn Lys Ser Gly Ile
    370                 375                 380

Ser Phe Glu Gly Ser Lys Ser Ser Phe Gly Gly Ala Ile Ala Cys Gly
385                 390                 395                 400

Asn Phe Ser Ser Glu Asn Asn Ser Ser Ala Leu Gly Ser Ile Asp Ile
                405                 410                 415

Ser Asn Asn Leu Gly Asp Ile Ser Phe Leu Arg Thr Leu Cys Thr Thr
            420                 425                 430

Ser Asp Leu Gly Gln Thr Asp Tyr Gln Gly Gly Gly Ala Leu Phe Ala
        435                 440                 445

Glu Asn Ile Ser Leu Ser Glu Asn Ala Gly Ala Ile Thr Phe Lys Asp
    450                 455                 460

Asn Ile Val Lys Thr Phe Ala Ser Asn Gly Lys Met Leu Gly Gly Gly
465                 470                 475                 480

Ala Ile Leu Ala Ser Gly Asn Val Leu Ile Ser Lys Asn Ser Gly Glu
                485                 490                 495

Ile Ser Phe Val Gly Asn Ala Arg Ala Pro Gln Ala Ile Pro Thr Arg
            500                 505                 510

Ser Ser Asp Glu Leu Ser Phe Gly Ala Gln Leu Thr Gln Thr Thr Ser
        515                 520                 525

Gly Cys Ser Gly Gly Gly Ala Leu Phe Gly Lys Glu Val Ala Ile Val
    530                 535                 540

Gln Asn Ala Thr Val Val Phe Glu Gln Asn Arg Leu Gln Cys Gly Glu
545                 550                 555                 560

Gln Glu Thr His Gly Gly Gly Gly Ala Val Tyr Gly Met Glu Ser Ala
                565                 570                 575

Ser Ile Ile Gly Asn Ser Phe Val Arg Phe
                580                 585

<210>  18
<211>  645
<212>  PRT
<213>  C. muridarum

<400>  18

Met Pro Pro Gly Ile Tyr Asp Gly Thr Thr Leu Thr Ala Pro Phe Pro
1               5                   10                  15

Tyr Thr Val Ile Gly Asp Pro Arg Gly Thr Lys Val Thr Ser Ser Gly
            20                  25                  30

Ser Leu Glu Leu Lys Asn Leu Asp Asn Ser Ile Ala Thr Leu Pro Leu
        35                  40                  45

Ser Cys Phe Gly Asn Leu Leu Gly Asn Phe Thr Ile Ala Gly Arg Gly
    50                  55                  60

His Ser Leu Val Phe Glu Asn Ile Arg Thr Ser Thr Asn Gly Ala Ala
65                  70                  75                  80

Leu Ser Asn His Ala Pro Ser Gly Leu Phe Val Ile Glu Ala Phe Asp
                85                  90                  95

Glu Leu Ser Leu Leu Asn Cys Asn Ser Leu Val Ser Val Val Pro Gln
            100                 105                 110

Thr Gly Gly Thr Thr Thr Ser Val Pro Ser Asn Gly Thr Ile Tyr Ser
        115                 120                 125

Arg Thr Asp Leu Val Leu Arg Asp Ile Lys Lys Val Ser Phe Tyr Ser
    130                 135                 140

Asn Leu Val Ser Gly Asp Gly Gly Ala Ile Asp Ala Gln Ser Leu Met
145                 150                 155                 160

Val Asn Gly Ile Glu Lys Leu Cys Thr Phe Gln Glu Asn Val Ala Gln
                165                 170                 175

Ser Asp Gly Gly Ala Cys Gln Val Thr Lys Thr Phe Ser Ala Val Gly
            180                 185                 190

Asn Lys Val Pro Leu Ser Phe Leu Gly Asn Val Ala Gly Asn Lys Gly
        195                 200                 205

Gly Gly Val Ala Ala Val Lys Asp Gly Gln Gly Ala Gly Gly Ala Thr
    210                 215                 220

Asp Leu Ser Val Asn Phe Ala Asn Asn Thr Ala Val Glu Phe Glu Gly
225                 230                 235                 240

Asn Ser Ala Arg Ile Gly Gly Gly Ile Tyr Ser Asp Gly Asn Ile Ser
                245                 250                 255

Phe Leu Gly Asn Ala Lys Thr Val Phe Leu Ser Asn Val Ala Ser Pro
            260                 265                 270

Ile Tyr Val Asp Pro Ala Ala Ala Gly Gly Gln Pro Pro Ala Asp Lys
        275                 280                 285

Asp Asn Tyr Gly Asp Gly Gly Ala Ile Phe Cys Lys Asn Asp Thr Asn
    290                 295                 300

Ile Gly Glu Val Ser Phe Lys Asp Glu Gly Val Val Phe Phe Ser Lys
305                 310                 315                 320

Asn Ile Ala Ala Gly Lys Gly Gly Ala Ile Tyr Ala Lys Lys Leu Thr
                325                 330                 335

Ile Ser Asp Cys Gly Pro Val Gln Phe Leu Gly Asn Val Ala Asn Asp
            340                 345                 350

Gly Gly Ala Ile Tyr Leu Val Asp Gln Gly Glu Leu Ser Leu Ser Ala
        355                 360                 365

Asp Arg Gly Asp Ile Ile Phe Asp Gly Asn Leu Lys Arg Met Ala Thr
    370                 375                 380

Gln Gly Ala Ala Thr Val His Asp Val Met Val Ala Ser Asn Ala Ile
385                 390                 395                 400

Ser Met Ala Thr Gly Gly Gln Ile Thr Thr Leu Arg Ala Lys Glu Gly
                405                 410                 415

Arg Arg Ile Leu Phe Asn Asp Pro Ile Glu Met Ala Asn Gly Gln Pro
            420                 425                 430

Val Ile Gln Thr Leu Thr Val Asn Glu Gly Glu Gly Tyr Thr Gly Asp
        435                 440                 445

Ile Val Phe Ala Lys Gly Asp Asn Val Leu Tyr Ser Ser Ile Glu Leu
    450                 455                 460

Ser Gln Gly Arg Ile Ile Leu Arg Glu Gln Thr Lys Leu Leu Val Asn
465                 470                 475                 480

Ser Leu Thr Gln Thr Gly Gly Ser Val His Met Glu Gly Gly Ser Thr
                485                 490                 495

Leu Asp Phe Ala Val Thr Thr Pro Pro Ala Ala Asn Ser Met Ala Leu
            500                 505                 510

Thr Asn Val His Phe Ser Leu Ala Ser Leu Leu Lys Asn Asn Gly Val
        515                 520                 525

Thr Asn Pro Pro Thr Asn Pro Pro Val Gln Val Ser Ser Pro Ala Val
    530                 535                 540

Ile Gly Asn Thr Ala Ala Gly Thr Val Thr Ile Ser Gly Pro Ile Phe
545                 550                 555                 560

Phe Glu Asp Leu Asp Glu Thr Ala Tyr Asp Asn Asn Gln Trp Leu Gly
                565                 570                 575

Ala Asp Gln Thr Ile Asp Val Leu Gln Leu His Leu Gly Ala Asn Pro
            580                 585                 590

Pro Ala Asn Ala Pro Thr Asp Leu Thr Leu Gly Asn Glu Ser Ser Lys
        595                 600                 605

Tyr Gly Tyr Gln Gly Ser Trp Thr Leu Gln Trp Glu Pro Asp Pro Ala
    610                 615                 620

Asn Pro Pro Gln Asn Asn Ser Tyr Met Leu Lys Ala Ser Trp Thr Lys
625                 630                 635                 640

Thr Gly Tyr Asn Pro
                645

<210>  19
<211>  663
<212>  PRT
<213>  C. muridarum

<400>  19

Gln Val Leu Thr Pro Asn Val Ile Ile Pro Phe Lys Gly Asp Asp Ile
1               5                   10                  15

Tyr Leu Asn Gly Asp Cys Val Phe Ala Ser Ile Tyr Ala Gly Ala Glu
            20                  25                  30

Gln Gly Ser Ile Ile Ser Ala Asn Gly Gln Asn Leu Thr Ile Val Gly
        35                  40                  45
Gln Asn His Thr Leu Ser Phe Thr Asp Ser Gln Gly Pro Ala Leu Gln
    50                  55                  60
Asn Cys Ala Phe Ile Ser Ala Glu Glu Lys Ile Ser Leu Arg Asp Phe
65                  70                  75                  80
Ser Ser Leu Leu Phe Ser Lys Asn Val Ser Cys Gly Glu Lys Gly Met
                85                  90                  95
Ile Ser Gly Lys Thr Val Ser Ile Ser Gly Gly Asp Ser Ile Val Phe
            100                 105                 110
Lys Asp Asn Ser Val Gly Tyr Ser Ser Leu Pro Ser Val Gly Gln Thr
        115                 120                 125
Pro Thr Thr Pro Ile Val Gly Asp Val Leu Lys Gly Ser Ile Phe Cys
    130                 135                 140
Val Glu Thr Gly Leu Glu Ile Ser Gly Val Lys Lys Glu Leu Val Phe
145                 150                 155                 160
Asp Asn Thr Ala Gly Asn Phe Gly Ala Val Phe Cys Ser Arg Ala Ala
                165                 170                 175
Gln Gly Asp Thr Thr Phe Thr Val Lys Asp Cys Lys Gly Lys Ile Leu
            180                 185                 190
Phe Gln Asp Asn Val Gly Ser Cys Gly Gly Gly Val Ile Tyr Lys Gly
        195                 200                 205
Glu Val Leu Phe Gln Asp Asn Glu Gly Glu Met Leu Phe Arg Gly Asn
    210                 215                 220
Ser Ala His Asp Asp Leu Gly Ile Leu Asp Ala Asn Pro Gln Pro Pro
225                 230                 235                 240
Thr Glu Val Gly Gly Gly Gly Gly Val Ile Cys Thr Pro Glu Lys Thr
                245                 250                 255
Val Thr Phe Lys Gly Asn Lys Gly Pro Ile Thr Phe Asp Tyr Asn Phe
            260                 265                 270
Ala Lys Gly Arg Gly Gly Ala Ile Gln Ser Gln Thr Phe Ser Leu Val
        275                 280                 285

Ala Asp Ser Ala Val Val Phe Ser Asn Asn Thr Ala Glu Lys Gly Gly
    290                 295                 300

Gly Ala Ile Tyr Ala Leu Glu Val Asn Val Ser Thr Asn Gly Gly Ser
305                 310                 315                 320

Ile Leu Phe Glu Gly Asn Arg Ala Ser Glu Gly Gly Ala Ile Cys Val
                325                 330                 335

Ser Glu Pro Ile Ala Ala Asn Asn Gly Gly Leu Thr Leu His Ala Ala
            340                 345                 350

Asp Gly Asp Ile Ile Phe Ser Lys Asn Met Thr Ser Asp Arg Pro Gly
        355                 360                 365

Glu Arg Ser Ala Ile Arg Ile Leu Asp Ser Gly Thr Asn Val Ser Leu
    370                 375                 380

Asn Ala Ser Gly Ala Ser Lys Met Ile Phe Tyr Asp Pro Val Val Gln
385                 390                 395                 400

Asn Asn Pro Ala Thr Pro Pro Thr Gly Thr Ser Gly Glu Ile Lys Ile
                405                 410                 415

Asn Glu Ser Gly Ser Gly Ser Val Val Phe Thr Ala Glu Thr Leu Thr
            420                 425                 430

Pro Ser Glu Lys Leu Asn Val Ile Asn Ala Thr Ser Asn Phe Pro Gly
        435                 440                 445

Asn Leu Thr Val Ser Ser Gly Glu Leu Val Val Thr Lys Gly Ala Thr
    450                 455                 460

Leu Thr Val Gly Asn Ile Thr Ala Thr Ser Gly Arg Val Thr Leu Gly
465                 470                 475                 480

Ser Gly Ala Ser Leu Ser Ala Val Ala Gly Thr Ala Gly Thr Cys Thr
                485                 490                 495

Val Ser Lys Leu Gly Ile Asp Leu Glu Ser Phe Leu Val Pro Thr Tyr
            500                 505                 510

Glu Thr Ala Lys Leu Gly Ala Asp Thr Thr Val Ala Val Asn Asn Asn
        515                 520                 525

Pro Thr Leu Asp Leu Val Met Ala Asn Glu Thr Glu Met Tyr Asp Asn

530                 535                 540

Pro Leu Phe Met Asn Ala Val Thr Ile Pro Phe Val Thr Leu Val Ser
545                 550                 555                 560

Leu Gln Thr Thr Gly Gly Val Thr Thr Ser Ala Val Thr Leu Asn Asn
                565                 570                 575

Ala Asp Thr Ala His Tyr Gly Tyr Gln Gly Ser Trp Ser Ala Asp Trp
            580                 585                 590

Arg Arg Pro Pro Leu Ala Pro Asp Pro Ser Gly Met Thr Pro Leu Asp
        595                 600                 605

Lys Ser Asn Thr Leu Tyr Val Thr Trp Arg Pro Ser Ser Asn Tyr Gly
    610                 615                 620

Val Tyr Lys Leu Asp Pro Gln Arg Arg Gly Glu Leu Val Pro Asn Ser
625                 630                 635                 640

Leu Trp Val Ser Gly Ser Ala Leu Arg Thr Phe Thr Asn Gly Leu Lys
                645                 650                 655

Glu His Tyr Val Ser Arg Asp
            660

<210>  20
<211>  433
<212>  PRT
<213>  C. muridarum

<400>  20
   Met Arg Thr Leu Ser Ile Ser Met Leu Ile Leu Ala Leu Ser Cys Gly
   1               5                   10                  15
   Glu Asn Thr Cys Leu Cys Ala Ala Asp Ser Pro Lys Ala Lys Val Asp
               20                  25                  30
   Ala Ser Ile Gly Asn Gly Ala Ser Phe Ser Pro Phe Thr Gly Glu Ile
           35                  40                  45
   Lys Gly Asn Arg Val Arg Leu Arg Leu Ala Pro His Thr Asp Ser Ser
       50                  55                  60
   Ile Ile Lys Glu Leu Ser Lys Gly Asp Cys Leu Ala Val Leu Gly Glu
   65                  70                  75                  80
   Ser Lys Asp Tyr Tyr Val Val Ala Ala Pro Glu Gly Val Arg Gly Tyr
   85 90 95
   Val Phe Arg Thr Phe Val Leu Asp Asn Val Ile Glu Gly Glu Lys Val
   100 105 110
   Asn Val Arg Leu Glu Pro Ser Thr Ser Ala Pro Ile Leu Ala Arg Leu
   115 120 125
   Ser Lys Gly Thr Val Val Lys Thr Leu Gly Ala Ala Gln Gly Lys Trp
   130 135 140
   Val Glu Ile Ala Leu Pro Lys Gln Cys Val Phe Tyr Val Ala Lys Asn
   145 150 155 160
   Phe Val Lys Asn Val Gly Ala Leu Glu Leu Tyr Asn Gln Lys Glu Gly
   165 170 175
   Gln Lys Lys Ile Ala Leu Asp Leu Leu Asn Ser Ala Met Ser Phe Ala
   180 185 190
   Asp Ala Glu Leu Gln Lys Lys Val Glu Asp Ile Asp Leu Asp Ala Ile
   195 200 205
   Tyr Lys Lys Met Asn Leu Ala Gln Ala Glu Glu Phe Lys Asp Val Pro
   210 215 220
   Gly Leu Gln Pro Leu Val Gln Lys Ala Leu Glu Arg Val Gln Glu Ala
   225 230 235 240
   Phe Leu Ala Lys Ser Leu Glu Lys Gly Ser His Lys Thr Val Glu Ser
   245 250 255
   Tyr Lys Pro Val Glu Thr Gln Ala Gln Leu Gln Pro Gln Arg Gln Val
   260 265 270
   Ile Glu Glu Lys Asn Val Ser Val Val Pro Glu Ala Pro Val Leu Ser
   275 280 285
   Gln Val Glu Glu Pro Lys Ser Val Leu Thr Ser Ser Ser Glu Val Glu
   290 295 300
   Pro Leu Gln Asp Val Gly Pro Ile Lys Gly Ser Leu Leu Ser His Tyr
   305 310 315 320
   Ile Arg Lys Lys Gly Phe Val Lys Thr Ser Pro Val Val Glu Gly Arg
   325 330 335
   Glu Ser Phe Glu Arg Ser Leu Phe Glu Val Trp Val Asn Leu Gln Pro
               340                 345                 350
   Glu Glu Ile Arg Asn Gly Leu Thr Met Glu Ser Phe Tyr Arg Asp Glu
           355                 360                 365
   Gln Lys Lys Lys Arg Val Leu Thr Gly Glu Leu Glu Val Tyr Pro His
       370                 375                 380
   Ile Val Lys Asn Asn Pro Gly Asp Tyr Leu Leu Lys Asn Gly Glu Asp
   385                 390                 395                 400
   Val Val Ala Phe Val Tyr Ala Thr Ser Ile Asp Leu Ser Lys Trp Leu
                   405                 410                 415
   Gly Lys Arg Val Val Leu Glu Cys Val Ser Arg Pro Asn Asn His Phe
               420                 425                 430
   Ala

## Description

The present invention relates in a first aspect to an immunogenic composition comprising at least three *Chlamydia ssp. surface* antigens selected from the group of Pmp A, Pmp D, Pmp G, Pmp H in combination with the antigen Ctad1. Further, the present invention relates to the immunogenic composition comprising CDN as adjuvant, in particular, c-diAMP. Moreover, a pharmaceutical composition comprising the immunogenic composition according to the present invention is provided as well as a vaccine comprising said immunogenic composition. The vaccine is particularly useful in eliciting an immune response against *Chlamydia ssp.* in an animal, in particular, for use in treating or preventing the infection by *Chlamydia ssp.* The vaccine, pharmaceutical composition or immunogenic composition may be administered mucosally, preferably is administered at least three times.

### Prior art

*Chlamydiae* are Gram-negative, intracellular bacteria with a unique reproductive cycle: infective elementary bodies (EBs) induce their uptake into mucosal cells where they remain in inclusions. In this niche, they become metabolically active reticulate bodies and divide until hundreds of new infectious EBs are produced and released. *Chlamydiae* cause infections of urogenital, respiratory and gastrointestinal tract, and the eye. *Chlamydia trachomatis* (*Ctr*) comprises 19 serovars based on the major outer membrane protein (MOMP), and over 60 genotypes.

Serovars D-K ('genital tract biovar') and L1-L3 ('lymphogranuloma venereum biovar') lead to infections of urethra or cervix uteri. With an estimated prevalence of 4.2% in 14 to 49 year old men and women worldwide, *Ctr* is the most reported bacterium causing sexually-transmitted disease (STD). According to WHO, there were 130 million new cases in 2015. Acute genital *Ctr* infections lead to few physical complaints. Hence, 80% remain unrecognized and untreated. PCR studies suggest that 4.4% of sexually active 17 year-old female teenagers and 4.5% of all 18-19 year-old women in Germany are latently infected with *Ctr.* Partially due to different serovars, *Ctr* can repetitively infect the same person (reviewed in Phillips S. et al. (2019) Front. Microbiol. 10:70)*.* It can ascend to the upper genital tract resulting in inflammation of the fallopian tube or ovary, or other painful and difficult to treat pelvic inflammatory diseases. Ectopic pregnancy is one of the feared sequelae of tissue damage. More important, it has been estimated that 1 out of 200 urogenital infections leads to permanent tubal infertility. Additionally, there is a relevant financial burden for society caused by often-unsuccessful therapeutic attempts to satisfy later the wish to have children. Pregnant women can transmit *Ctr* to newborns in the birth channel leading to conjunctivitis and pneumonia, which do not readily respond to therapy. Men (with a certain genetic background) in particular can suffer from reactive arthritis. Up to years, persistent viable *Ctr* with reduced metabolism are detectable in the synovia of the affected joints in this rheumatic disease. Additionally, there is an ongoing discussion about a potential link between *Ctr* infections and urogenital tumors. In developing countries, this STD is even more frequent, and to manage the disease and to avoid its sequelae, routine PCR screening of young woman is impracticable. Even in industrialized countries, only screening for *Ctr*-DNA during pregnancy has been successful. Attempts to change human risk behavior and to decrease infection rates by information-campaigns and the opportunity for all woman ≤25 years for an annual PCR screening paid by the health insurance remained ineffective.

The serovars A-C of *Ctr* ('trachoma biovar') are responsible for trachoma. According to the WHO Global Health Observatory 2019, this neglected tropical disease remains a public health problem in 43 countries. It is responsible for partial or total visual impairment of 1.8 million people. In 2017, 83.5 million people received antibiotics for trachoma, and 231,000 people received surgery for its late blinding stage, trachomatous trichiasis. The WHO aims to eliminate trachoma by 2020. Apparently, with the so far applied SAFE Strategy (Surgery, Antibiotics, Facial cleanliness, Environmental improvement) alone, it will be impossible to achieve this ambitious, but highly unrealistic aim.

However, there is still no vaccine available. Most attempts of vaccine development have been unsuccessful, either due to missing protection or even due to increased immunopathology during a subsequent chlamydial infection (reviewed in e.g. Gottlieb SL and Johnston C (2017) Curr Opin Infect Dis. 30:77-86). According to the review "Seventy Years of Chlamydia Vaccine Research - Limitations of the Past and Directions for the Future" (Phillips S. *et al.* see above), 78 studies have been performed between 1949 and 2017. Its main conclusion is that"... no single antigen type or target, adjuvant, or route of administration has been established as a clear front-runner for effective vaccination. Extensive mouse model trials indicate that whole cell antigenic targets induce an effective response, protecting from disease and reducing shedding rates. However, replication of these results using more commercially acceptable antigenic preparations has proven difficult".

Although studies on Ctr vaccination have been conducted, most of them had some adverse side effects not desired for application in human. For example, Stary et al., 2015, Science 348 (6241 :aaa8205) applied UV-inactivated EBs alone or with a commonly used adjuvants, 5 to 10 times more *Chlamydia* were found after a genital challenge infection in the uterus of vaccinated as compared to non-vaccinated mice. That is, inactivated or killed *Chlamydia* as such are not suitable for vaccination. This is most described already in the 1960. Thus, safety concerns and challenges manufacturing a whole cell *Chlamydia* vaccine to industry standards are major obstacles and has prompted the search for a subunit vaccine.

Therefore, most alternative approaches are based on recombinant or purified chlamydial surface proteins, particularly the major outer membrane protein (MOMP) and polymorphic membrane proteins (Pmp's). According to the overview by Phillips S. *et al.,* see *above* from 2019: "To date, the MOMP has emerged as the most suitable substitute for whole cell targets and its delivery as a combined systemic and mucosal vaccine is most effective." That refers in particular to a complex vaccine combining immunogenic parts of several MOMP molecules from different serovars in order to cover the most frequent ones combining s.c. and i.n. administration (Wern JE, et. al., (2017) Front. Immunol. 8:569) A phase-1-clinical trial (https://clinicaltrials.gov/ct2/show/ NCT03926728) has been recently finished. However, without loss of function MOMP can mutate and is subject to immune selective pressure and recombination as demonstrated by the existing various genotypes and serovars. Population-wide vaccination with parts of this molecule - if successful - might raise such pressure leading to the selection of *Ctr* serovars, which are not targeted by the vaccine and of *Ctr* MOMP mutants who escape the defense caused by vaccine-induced antibodies or T cells.

The nine Pmp proteins A to I form the largest protein family in Ctr. Recombinant production of the native form of the Pmp proteins have been described. rPmp proteins act as adhesins, and proved that all *Ctr* Pmps are essential for infection (Becker E, Hegemann JH (2014) Microbiologyopen 3(4):544-56. doi: 10.1002/mbo3.186.). Pmps are large proteins (∼ 100 to 150 kDa) and share autotransporter characteristics with a short N-terminal Sec signal sequence (24 to 50 amino acids, aa), a large passenger domain (PD, ∼ 650 to 1200 aa) and a C-terminal β-barrel (∼ 300 aa) for outer membrane translocation. Evidence suggests that Pmp proteins form homo- and heteromeric oligomers. Antibodies to PmpD neutralize all *Ctr* serovars *in vitro* (Crane DD, et. al., (2006) Proc Natl Acad Sci USA. 103(6): 1894-9.). Pmp antigens lead to protection in various animal systems. Vaccination of mice with short PD protein fragments from PmpE, F, G and H from *C*. *muridarum* (*Cmu,* formerly called mouse pneumonitis agent of *Ctr*)*,* with DDA/MPL as adjuvant, confers protection against a *Cmu* vaginal challenge (Yu H, et. al., (2012) Infect Immun. 80(4): 1510-1518. doi: 10.1128/IAI.06338-11). Moreover, PmpE, F, G and H fragments plus MOMP from *Ctr* D protect against a *Ctr* challenge in mice (Karunakaran KP, et. al., (2015) Vaccine. 33(18):2159-66. doi: 10.1016/j.vaccine.2015.02.055. Epub 2015 Mar 1.).

Pal S, et. al., (2017) Vaccine 35, DOI: 10.1016/j.vaccine.2017.03.070 provided in three Pmp-based cross-species vaccine trials recombinant Pmp proteins. Based on the high protein identity between *Ctr* serovars and *Cmu,* short protein fragments from the PD from all nine Ctrserovar E Pmps were tested in mice using s.c. application and CpG-1826 and Montanide ISA 720 VG as adjuvants. Indeed, this results in limited cross-species protection against a subsequent *Cmu* infection with Pmp C protecting best followed by PmpG, E or H. A PD fragment from PmpC from *Ctr* E applied s.c. and i.m. also cross-protects against a challenge with *C. caviae* in the guinea pig. Similarly, partial cross-protection was also observed for a PmpA PD fragment from *Ctr* E plus CpG-ODN 1826 adjuvant against a subsequent *Cmu* infection; yet, here also adjuvant-induced immunopathology upon challenge of the genital tract was reported (Muller T, et., al. (2017) Vaccine 35, DOI: 10.1016/j.vaccine.2017.04.017). Thus, Pmp proteins can clearly protect against a *Ctr* infection and show even partial cross-species protection.

Stallmann et al., 2016, Cell Microbiol, 18(5), 761-775 describes Ctad1 of Chlamydia trachomatis as a new adhesin and invasion allowing binding to human cells.

Hence, there is still a need to develop a vaccine against Ctr preventing urogenital infection with its sequelae, as well as for preventing trachoma.

### Brief description of the present invention

The present invention relates in a first aspect to an immunogenic composition comprising at least three *Chlamydia ssp.* surface antigens selected from the group of polymorphic membrane protein PmpA, PmpD, PmpG and PmpH in combination with the antigen Ctad1.

In a further aspect, the present invention relates to the immunogenic composition comprising the mentioned *Chlamydia ssp.* surface antigens according to the present invention in combination with an adjuvant, in particular, a cyclic dinucleotide (CDN) adjuvant including c-di-AMP.

Furthermore, the present invention provides a pharmaceutical composition comprising the immunogenic composition according to the present invention. In an embodiment, the immunogenic composition or the pharmaceutical composition is in form of a vaccine. The vaccine may be used in preventing or treating an infection by *Chlamydia ssp.* in an animal including humans.

Moreover, the present invention provides methods for treating Chlamydia infection and sequelae of the same including trachoma, infertility and respiratory tract diseases, the vaccine is particularly suitable for mucosal administration.

### Brief description of the drawings

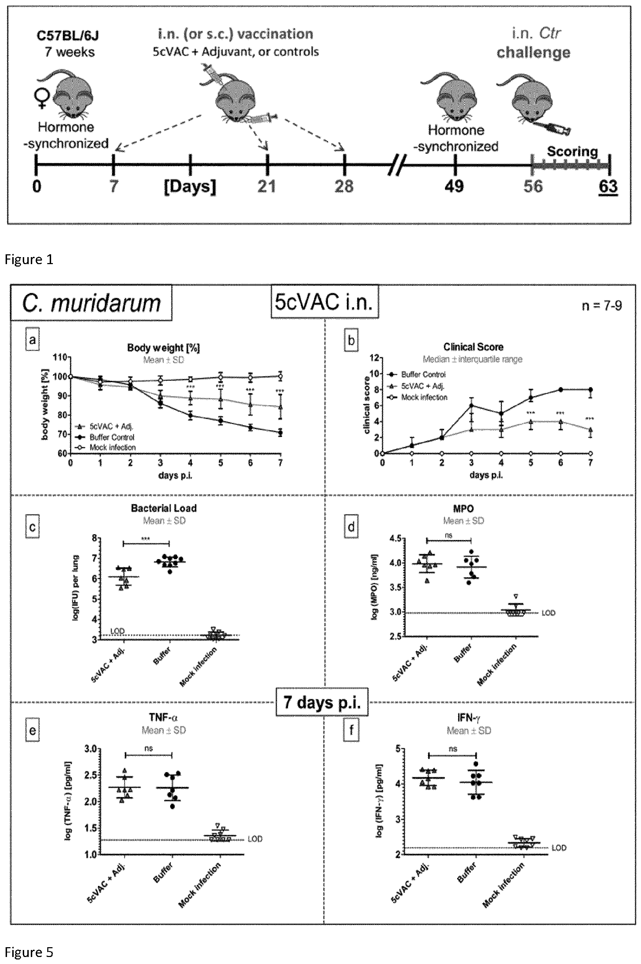

Figure 1: Experimental setup of the mouse vaccination - *Chlamydia trachomatis* lung challenge infection model

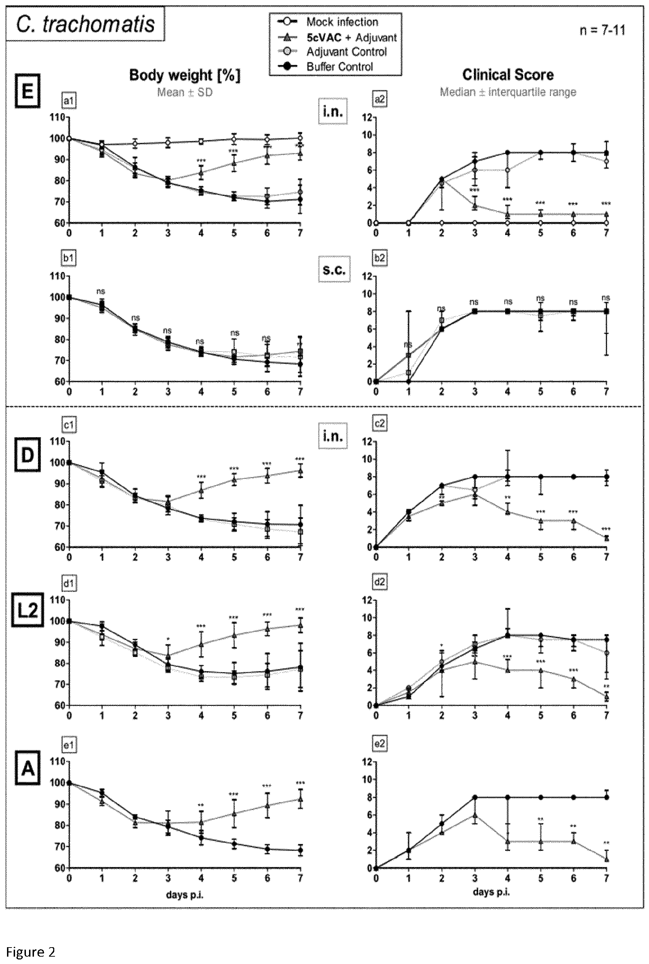

Figure 2: Intranasal (i.n.) vaccination with 5cVAC improves loss of body weight (a-e 1) and clinical score (a-e 2) in lung challenge infection with serovar E (a1/a2), D, L2 (c1/c2), or A (e1/e2) of *Chlamydia trachomatis.* In contrast, subcutaneous (s.c.) vaccination is less effective, as demonstrated for Serovar E (b1/b2) which represents best the antigens the vaccine is consisting of.

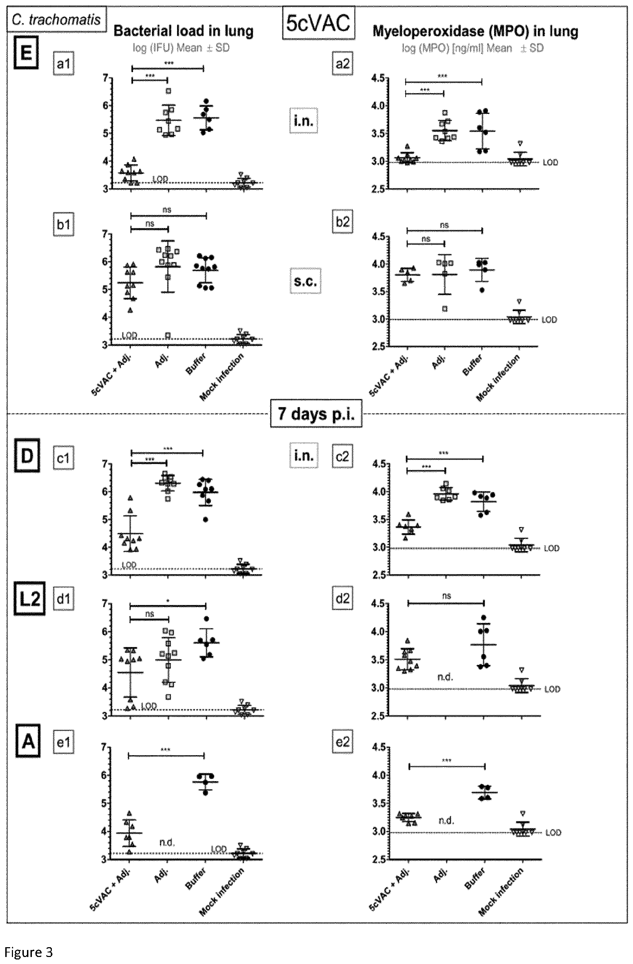

Figure 3: Intranasal (i.n.) vaccination with 5cVAC improves bacterial clearance and leads to decreased levels of the granulocyte marker MPO in the lung after i.n. challenge infection with different serovars of *Chlamydia trachomatis* as determined on day 7 p.i.. In contrast, subcutaneous (s.c.) vaccination is less effective, as demonstrated for Serovar E which represents best the antigens 5cVAC is consisting of.

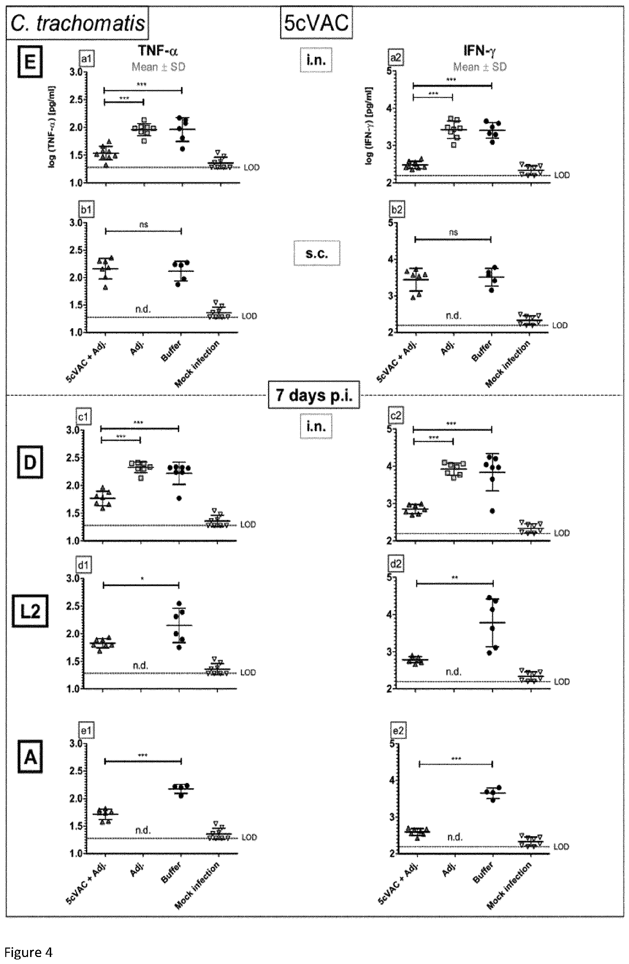

Figure 4: Intranasal (i.n.) vaccination with 5cVAC leads to decreased levels of the key cytokines TNF-alpha and IFN-gamma in lung challenge infection with different serovars of *Chlamydia trachomatis* determined on day 7 p.i.. In contrast, subcutaneous (s.c.) vaccination is less effective, as demonstrated for Serovar E.
Figure 5: Intranasal vaccination with 5cVAC diminishes *Chlamydia muridarum-*induced loss of body weight, the increase in clinical score and the bacterial load on day 7 after challenge infection.

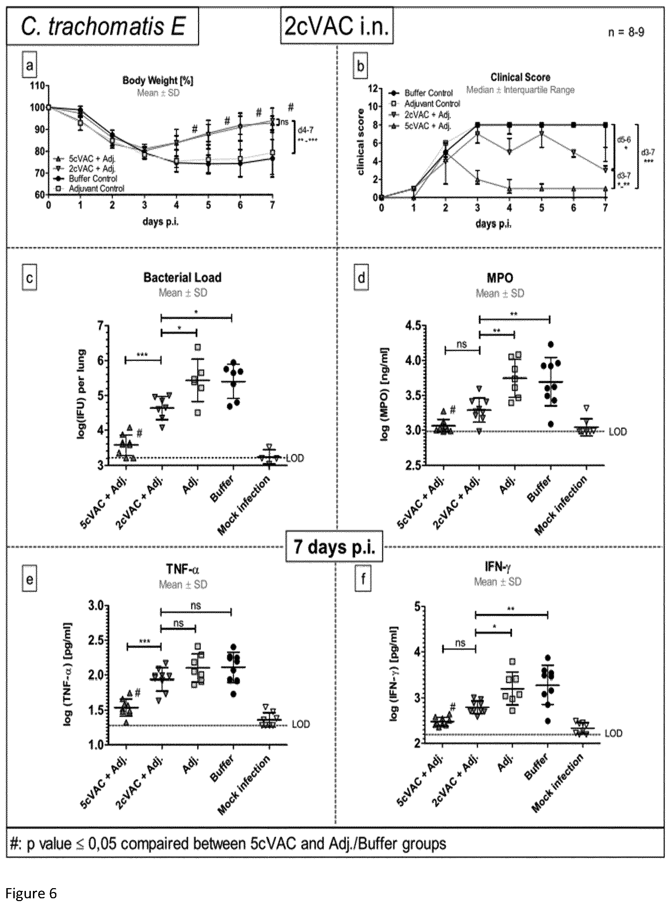

Figure 6: Intranasal vaccination with two-component 2cVAC (PmpD and Ctad1) is still effective but with a smaller degree pf protection as compared to 5cVAC in lung challenge infection with Ctr E.

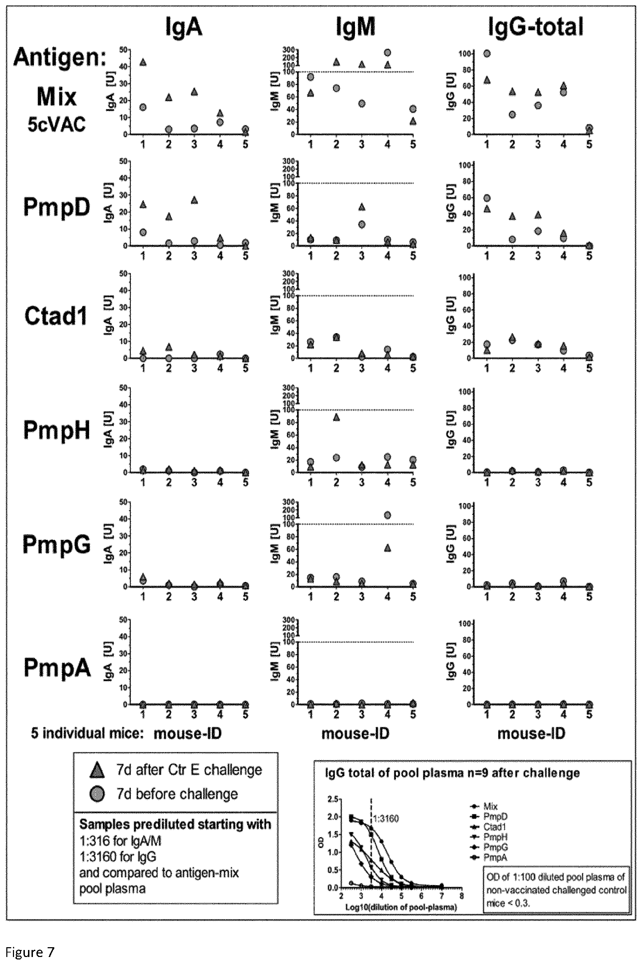

Figure 7: IgA, IgM and total IgG antibody response profiles towards the 5cVAC antigen-mix or the single vaccine components in mouse plasma of 5 individual mice after vaccination with 5cVAC, 7 days before or after Ctr E challenge, respectively. Moreover, less limiting anti-5cVac IgG of pool-plasma of n = 9 mice obtained 7 days p.i. was also analyzed.

### Detailed description of the present invention

In a first aspect, the present invention relates to an immunogenic composition comprising at least three *Chlamydia ssp.* surface antigens selected from the group of polymorphic membrane protein PmpA, PmpD, PmpG and PmpH in combination with the antigen Ctad1.

The present inventors recognized that with an immunogenic composition with the *Chlamydia ssp.* surface antigens selected from the group of PmpA, PmpD, PmpG and PmpH in combination with the antigen Ctad1 of *Chlamydia ssp.,* good vaccination results against Chlamydia can be achieved.

Namely, at least three of the mentioned surface antigens are suitable for elicit respective immune response against Chlamydia. In an embodiment, the at least three *Chlamydia ssp.* surface antigens are Ctad1 in combination with two out of the four Pmp antigen. PmpA, PmpD, PmpG and PmpH. In an embodiment, at least four *Chlamydia ssp.* surface antigens are present in the immunogenic composition. In an embodiment, one of said at least four antigens is the Ctad1 antigen.

In an embodiment, the immunogenic composition according to the present invention comprises at least the *Chlamydia ssp.* surface antigens of PmpA, PmpD, PmpG, PmpH and Ctad1.

As used herein, the term "comprise" or "contain" includes the embodiment of "consist of".

Typically, the antigens are present in the immunogenic composition in form of recombinant polypeptides. For example, the antigens are PmpA of SEQ ID No. 1, PmpD of SEQ ID No. 2, PmpG of SEQ ID No. 3, PmpH of SEQ ID No. 4, and Ctad1 of SEQ ID No. 5 derived from the respective proteins of *Chlamydia trachomatis* serotype E, PmpA of SEQ ID No. 6, PmpD of SEQ ID No. 7, PmpG of SEQ ID No. 8, PmpH of SEQ ID No. 9, and Ctad1 of SEQ ID No. 1 0 derived from the respective proteins of *Chlamydia trachomatis* serotype B, PmpA of SEQ ID No. 11, PmpD of SEQ ID No. 12, PmpG of SEQ ID No. 13, PmpH of SEQ ID No. 14, and Ctad1 of SEQ ID No. 5 1 derived from the respective proteins of *Chlamydia trachomatis* serotype LGV, PmpA of SEQ ID No. 16, PmpD of SEQ ID No. 17, PmpG of SEQ ID No. 18, Pmp H of SEQ ID No. 19, and Ctad1 of SEQ ID No. 20 derived from the respective proteins of C. *muridarum.*

That is, the antigens are recombinant Pmp proteins and Ctad1 protein comprising most of the amino acid sequence of the natural Pmp proteins and Ctad1 protein, respectively. In an embodiment, the Pmp proteins do not contain the beta-barrel and not the N-terminal signal sequence present in the native protein.

In an embodiment, the Ctad1 protein is the full length Ctad1 protein, e.g. ADH16779.1 (Ctr E), WP_012728031.1 (Ctr B), AGJ64368.2 (Ctr LGV), and AID37821.1 (*C. muridarum).* Further, the PmpA may be encoded by the sequence or a fragment of said sequence of the gene ADH17184.1 (Ctr E), WP_009871764 (Ctr B), YP_001654741.1 (Ctr LGV), and AID38210.1 (*C. muridarum*)*.* The PmpD protein is a polypeptide encoded by ADH17618.1 (Ctr E), WP_012728194.1 (Ctr B), YP_001654273.1 (Ctr LGV), and AID37735.1 (*C. muridarum*)*.* The PmpG is a polypeptide or fragment thereof encoded by ADH17684.1 (Ctr E), WP_011324914.1 (Ctr B), YP_001654335.1 (Ctr LGV), and AID37800.1 (*C. muridarum).* The PmpH antigen is a polypeptide or fragment thereof encoded by ADH17685.1 (Ctr E), WP_011324915.1 (Ctr B), YP_001654336.1 (Ctr LGV) and AID37801.1 (*C*. *muridarum*)*.*

The antigen according to the present invention may be a homologue of the native antigen. That is, the homologue may have a sequence identify of at least 90 %, at least 91 %, 92 %, 93 %, 94 %, 95 %, 96 %, 97 %, 98 %, 99 % of the respective native surface antigen present in the immunogenic composition according to the present invention. The homologues include said homologues with the specified identify which display similar antigenic activity as the respective polypeptide of said sequence of the surface antigen provided herein.

In an embodiment of the present invention, the *Chlamydia ssp.* surface antigens are derived or stemming from *Chlamydia trachomatis.* In an embodiment, the *Chlamydia trachomatis* is a *Chlamydia trachomatis* serotype E. Of course, other serotypes are suitable and include at least servars A, D, and L2 but also others such as B, C, F, G, H, J, K and L1 or L3.

In an embodiment of the present invention, the immunogenic composition according to the present invention comprise further an adjuvant.

Namely, in an embodiment, the adjuvant is a compound according to formula (I) 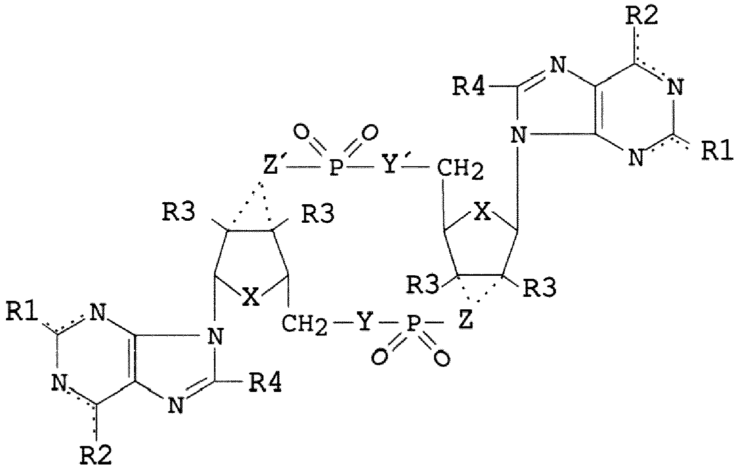 wherein
X is independently from one another S, N, O, CH₂;
Y, Y' is independently from one another NH, CH₂, O;
Z, Z' is independently from one another NH, CH₂, O;
R₁ is independently from one another hydrogen or O or absent;
R₂ is independently from one another NH₂, O, H, or a hydrogen;
R₃ is independently from one another absent if a covalent bond is present between the Z or Z' and the C atom, or is hydrogen, OH, halogen, a straight or branched C₁-C₆ alkyl group, or a straight or branched C₁-C₆ alkoxy group which may optionally be substituted;
R₄ is independently from one another hydrogen, halogen, or a straight or branched C₁-C₆ alkyl group, which may optionally be substituted;
... is a single or double bond;
or conjugates thereof, and salts or solvates thereof.

In an embodiment, the adjuvant, present in the immunogenic composition according to the present invention being a compound according to formula (I) wherein any one of the purine residue is an adenine, xanthine or hypoxanthine residue of combinations thereof. In an embodiment, both purine residues are adenine. That is, it is preferred that the cyclic di-nucleotide is a cyclic di-adenosine monophophate. For example, the c-diAMP is a 2,3'-c-diAMP or 3,3'-c-diAMP. Further, the adjuvant may be a biphosphatethioate analog of the c-diAMP. In an embodiment, the adjuvant of general formula (I) is a compound wherein R3 is a OH group, X is an oxygen and Y, Y', Z and Z' are oxygen. Further, the compound according to formula (I) representing an adjuvant present in the immunogenic composition is anyone of the claims 5 to 7 characterized in that the compound according to formula I is a cyclic bis (3'-5') diodenylic acid (CDA or c-diAMP, c-diIMP, c-IAMP, or a cyclic diAMP thiophosphate, c-diMP Thiophosphate and cAMP-IMP thiophosphate.

Generally, the term "adjuvant" means substances which are edit and/or co-formulated in an immunisation to the active antigen, i.e. the substance which provokes the desired immune response, in order to enhance or elicit or modulate the humoral and/or cell mediated (cellular) immune response against the active antigen: Preferably, the adjuvant, as described herein, is able to enhance or elicit the innate immune response.

The term "therapy" or "treatment" include vaccination of individual. Generally, the term "therapy" or "treatment" refers to a process that is intended to produce a beneficial change in the condition of an individual, like a mammal, e.g. a human, often refer to as a patient, or animal. A beneficial change can, for example, include one or more of: restoration of function, reduction of symptoms, limitation or retardation of progression of a disease, dis? or condition or prevention, limitation or retardation of detoriation of a patient's condition, disease or disorder. Such therapy usually encompasses the administration of an active principal, among others.

As used herein, the term "antigenic structure" or "antigen" refers to a structure capable of causing a cellular or humoral immune response. The antigenic structure, also known as epitope, is the part of the antigen, which is presented by the MHC or MHC like molecules. Further, the epitope or antigenic structure represents the part of an antigen recognized by antibodies direct against said antigen.

As used herein, the term "individual" or "subject" which is used herein interchangeably refers to a human be infected with Chlamydia or being of risk of Chlamydia infection. Preferably, the subject is a mammal, particularly preferred a human. The term "animal" include the term "human".

As used herein, the term "carrier" refers to a diluent, adjuvant, other than the adjuvant of formula (I) as described herein, excipient or vehicle.

With the term "which may be substitute" is meant the substitution with the straight or branched C1 to C6 alkyl group or is straight or branched C1 to C6 alkoxy group and/or with a halogen, hydroxyl group or carboxyl group.

As used herein, the term "CDN" or "cyclic di-nucleotide" refers to the compounds of formula (I) unless otherwise indicated.

The term "CDA" as used herein refer to cyclic di-AMP or c-di-AMP unless otherwise indicated. In an embodiment, CDA or c-di-AMP refer to the non-fluorinated and/or non-thionated CDA molecule.

The linkage between the phosphate and the sugar moiety may be via the C3 atom (3') or the C2 atom (2') of the sugar residue. That is, the compound according to formula (I) may be a 2', 2'-CDN, a 3', 2'-CDN, a 2', 3'-CDN, or a 3', 3'-CDN, like a 2', 2'- c-di-AMP, a 3', 2'- c-di-AMP, a 2', 3'- c-di-AMP, or a 3', 3'- c-di-AMP. In formula (I) the dotted lines between Z or Z' and the O at position 2 and 3 of the sugar moiety identifies that a covalent bond may be present or absent. Binding is either via the C2 or C3 atom of the sugar moiety. When binding of the phosphate moiety is via the C3 atom, R₃ is absent at C3 and is present at C2. That is, R₃ is present at the C-atom which is not linked with the phosphate moiety.

In an embodiment of the present invention, the immunogenic composition according to the present invention is for use in the prophylactic or therapeutic treatment of a *Chlamydia* infection and a disease caused by *Chlamydia* infection. In this connection, prophylactic treatment of a disease refers to embodiments of vaccination. That is, the immunogenic composition according to the present invention is for use in therapeutic or prophylactic vaccination of individuals.

In a further embodiment, the present invention relates to compounds according to the present invention for use as a mucosal adjuvant, in particular, for intranasal, sublingual, intra-NALT, oral, intra-rectal, intrafollicular, transfollicular, intrapulmonary, intrabronchial, intratekal, conjungtival, intravaginal or intraurethral administration, administration into the milk ducts of the breast or by inhalation.

Alternatively, the compound is for use according to the present invention for parenteral administration, in particular, for subcutaneous, intravenous, intradermal, intrafollicular or intramuscular administration.

The skilled person is well aware of suitable weight of administration as well as required dosage.

In a further embodiment, the present invention relates to a pharmaceutical composition comprising the immunogenic composition according to the present invention. In an embodiment, the immunogenic composition according to the present invention or the pharmaceutical composition according to the present invention is a vaccine. The vaccine according to the present invention as well as the pharmaceutical composition according to the present invention or the immunogenic composition according to the present invention are for use in eliciting an immune response against Chlamydia ssp., or recombinant thereof, in an animal, like a mammal, e.g. a human. The vaccine is particularly suitable for use in treating or preventing the infection by Chlamydia ssp. in an animal. Further, the vaccine is particularly suitable for use in preventing trachoma, infertility, reactive arthritis, conjunctivitis, keratoconjunctivitis, or visual impariments.

The pharmaceutical composition may be administered with a physiologically acceptable carrier to a patient, as described herein. In a specific embodiment, the term "pharmaceutically acceptable" means approved by a regulatory agency or other generally recognized pharmacopoeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium, carbonate, etc. Examples of suitable pharmaceutical carriers are described in "Remington's Pharmaceutical Sciences" by E.W. Martin (18th ed., Mack Publishing Co., Easton, PA (1990)). The pharmaceutical should suit the mode of administration.

Typically, pharmaceutically or therapeutically acceptable carrier is a carrier medium which does not interfere with the effectiveness of the biological activity of the active ingredients and which is not toxic to the host or patient.

"Therapeutically- or pharmaceutically-effective amount" as applied to the compositions of the instant invention refers to the amount of composition sufficient to induce a desired biological result. That result can be alleviation of the signs, symptoms, or causes of a disease, or any other desired alteration of a biological system. In the present invention, the result will typically involve an increase in the immunological responses to infection or a suppression of the responses to inflammatory processes.

In vitro assays may optionally be employed to help identifying optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems. Preferably, the pharmaceutical composition is administered directly or in combination with an adjuvant.

The term "administered" means administration of a therapeutically effective dose of the aforementioned pharmaceutical composition to an individual. By "therapeutically effective amount" is meant a dose that produces the effects for which it is administered. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.

Further, the pharmaceutical composition may contain additionally components, e.g. compounds like one or more anti-inflammatory molecules, anti-angiogenic molecules, cytotoxic molecules, immunomodulatory molecules, preferably chemokines, cytokines, CD40 ligand, costimulatory molecules or antibodies or mixtures thereof.

In addition, the pharmaceutical composition described herein may be characterized in that the components of the pharmaceutical composition are associated and/or incorporated and/or coated to a physical particle, preferably microparticle, nanoparticle, liposome, ISCOM, copolymer and/or biological particle, preferably bacterial ghosts.

The pharmaceuticals according to the present invention or the compounds for use according to the present invention may be used in methods applicable to both human therapy and veterinary applications, in particular, for human application. The compounds for the use as described herein having the desired therapeutic activity may be administered in a physiologically acceptable carrier to a patient, as described herein. Depending upon the manner of introduction, the compounds may be formulated in a variety of ways as discussed below. The concentration of therapeutically active compound in the formulation may vary from about 0.1-100 wt%. The agents as adjuvant(s) for use in therapeutic or prophylactic vaccination may be administered alone or in combination with other treatments.

Moreover, the vaccine according to the present invention, the pharmaceutical composition according to the present invention elicit an immune response against *Chlamydia trachomatis* or *Chlamydia muridarum,* e.g. it is for use in treating or preventing the infection by *Chlamydia trachomatis* or *Chlamydia muridarum.*

The vaccine is particularly suitable for use in humans.

In another aspect, the present invention relates to a kit containing the immunogenic composition according to the present invention and, optionally, the adjuvant as described herein. In particular, the kit according to the present invention comprising the immunogenic composition as defined herein in combination with the adjuvant, in particular, c-di-AMP, is described. Said kit is particularly for use in treating or preventing infection by *Chlamydia* ssp. in a subject. For example, the kit is for use in preventive vaccination against infection by *Chlamydia* ssp., thus, suitable for preventing or treating infections of urogenital, respiratory and gastrointestinal tract as well as the eye, like trachoma, infertility etc.

Moreover, the present invention relates to a method for vaccination of subjects for prevention or treating infection by *Chlamydia* ssp. Said vaccination is particularly a preventive vaccination against urogenital, respiratory and gastrointestinal tract, as well as eye disease including trachoma caused by *Chlamydia* infection. The method is preferably a preventive vaccination of an individual. In an embodiment, the vaccination is by mucosal rood including intra nasal or sublingual application of the immunogenic composition as described herein.

The method according to the present invention include administration of the immunogenic composition or the pharmaceutical composition or the vaccine as defined herein to a subject for eliciting an immune response against *Chlamydia* spp. or a component thereof. The method according to the present invention is particularly useful to present sequelae diseases in use by *Chlamydia* infection including trachoma, infertility, reactive arthritis, conjunctivitis, keratoconjunctivitis, or visual impairments.

These and other embodiments are disclosed and encompassed by the description and examples of the present invention. The present invention will be described further by the way of examples without limiting the same.

### Examples

### Experimental setup of the mouse vaccination - Chlamydia trachomatis lung challenge infection model

The cycle of 7 weeks old female C57BL/6J mice was synchronized by i.p. application of medroxyprogesterone acetate. To determine the effect of **vaccination,** the mice were treated with an identical volume of [antigens in buffer plus adjuvant], [buffer plus adjuvant], or [buffer alone] **at day 7, 21 and 28.** the vaccination experiments were performed with **5cVAC,** i.e. an equimolar mix of 5 purified recombinant antigens derived from serovar E of C. *trachomatis* (*Ctr* E): PmpA, PmpD, PmpG, Pmp H, Ctad1, namely, an immunogenic composition according to the present invention which are combined with the adjuvant C-di-Amp. One experiment was performed with **2cVAC,** an equimolar mix (and the same amount per antigen, with C-di-Amp) of only 2 of them: PmpD and Ctad1. Vaccination was mainly performed by the i.n. route. As indicated, in a few experiments the otherwise identical vaccine was s.c. applied.

On day 49, the cycle of the female mice was synchronized again by the hormone to diminish potential cycle-depending variation. On day 56, i.e. 4 weeks after the last booster vaccination, the 15 weeks old mice were challenged by i.n. application of Chlamydia. To induce a similar severity of the resulting lung disease, different amounts of inclusion forming units (IFU) of the genital serovars E, D, L2 and the ocular serovar A of *C*. *trachomatis* per mouse had to be used (1.3x10⁶ for E, 2x10⁶ for D, 4x10⁵ for L2, and 4x10⁶ for A). The mice were additionally challenged (6x10³ IFU) with the closely related species *C*. *muridarum* (former name: mouse pneumoniae strain of *C*. *trachomatis*)*.* Some control animals received mock-material instead. In order to reduce variation within experiments with a specific serovar or *C*. *muridarum,* as far as possible, mice of the vaccinated and the non-vaccinated control groups were housed in mixed cages, and handled in parallel. Figure 1 provides a scheme of the experimental setup. The results depicted in the following usually combine data obtained in 2 to 3 staggered, otherwise identical experiments.

Under close monitoring and using humane endpoint defining criteria, body weight and clinical score were determined daily. On day 63, i.e. day 7 of challenge infection and developing pneumonia, all mice were sacrificed painlessly for analysis: In one of their lungs the histological score was determined. In the homogenate of the other lung, chlamydial load (IFU), and the levels of the granulocyte marker myeloperoxidase (MPO) as well as of the key cytokines IFN-γ and TNF-α were quantified.
For statistical analysis the following tests have been used: Two-way ANOVA with Bonferroni post-test for comparison of body weight and Kruskal-Wallis test with Dunn's multiple comparison post-test (≥ 3 groups) or Mann-Whitney t-test (2 groups) for the clinical score. For bacterial load, MPO, IFN-γ and TNF-α, One-way ANOVA with Bonferroni's multiple comparison were used. In the majority of cases logarithmic transformation of parametric data was performed in order to accomplish Gaussian distribution. If not indicated otherwise, *-*** indicate statistical significances between the 5cVAC group and the buffer control group with p<0.05, <0.01, and <0.001, respectively.

All animal experiments were approved by the Lower Saxony state government and the corresponding authorities of the LAVES, and performed in accordance with the law of animal welfare used for experiments (TierSchVersV), with the German regulations of the GV-SOLAS for the protection of animal life and the FELASA.

### Intranasal (i.n.) vaccination with 5cVAC improves loss of body weight and clinical score in lung challenge infection with serovar E, D, L2, or A of Chlamydia trachomatis.

Following the protocol of the mouse "vaccination - chlamydial lung challenge model", n = 8-11 differently pretreated animals per group for Ctr E, D, and L2, and 7-9 mice per group for Ctr A, respectively, were i.n. infected or mock-infected. During the following 7 days body weight [%] (a1-e1) and clinical score (a2-e2) were assessed daily. Mice which were i.n. vaccinated with 5cVAC showed from day 4 on protection against the genital serovar E and cross-serovar protection against *Ctr* D, L2, and the ocular serovar A (a1/a2, c1-e1/c2-e2). In contrast, s.c. application of the identical vaccine, i.e. 5cVAC plus adjuvant had less effect on body weight or clinical score (b1 and b2), see figure 2. Experimental setup, statistical analysis and meaning of the * are described above.

### Intranasal (i.n.) vaccination with 5cVAC improves bacterial clearance and leads to decreased levels of the granulocyte marker MPO in the lung after i.n. challenge infection with different serovars of Chlamydia trachomatis as determined on day 7 p.i..

Following the protocol of the mouse "vaccination - chlamydial lung challenge model", differently pretreated animals were i.n. infected with *Ctr* E, D, L2, and A - or mock-infected, respectively. On day 7 of challenge, the amount of viable, infectious chlamydia (a1-e1) and the level of MPO (a2-e2) were determined in lung homogenate of the mice surviving that period see figure 3. Experimental setup, statistical analysis and meaning of the * are described above. LOD = limit of detection; n.d. = not determined; ns = not significant.

### Intranasal (i.n.) vaccination with 5cVAC leads to decreased levels of the key cytokines TNF-α and IFN-γ in lung challenge infection with different serovars of Chlamydia trachomatis determined on day 7 p.i

Following the protocol of the "vaccination - chlamydial lung challenge model", mice were either pre-treated with [antigens in buffer plus adjuvant], [buffer plus adjuvant], or [buffer alone]. On day 7 after i.n. challenge-infection with *Ctr* E, D, L2, and A, or mock-material, respectively, the animals were sacrificed for determination of the level of TNF-α (a1-e1) and IFN-γ (a2-e2) in their lung homogenate, see figure 4. Experimental setup, statistical analysis and meaning of the * are described above. LOD = limit of detection; n.d. = not determined; ns = not significant.

### Intranasal vaccination with 5cVAC diminishes Chlamydia muridarum-induced loss of body weight, the increase in clinical score and the bacterial load on day 7 after challenge infection.

Following the protocol of the "vaccination - chlamydial lung challenge model", mice were either pre-treated with [antigens in buffer plus adjuvant] or [buffer alone]. Per group, n = 7-9 differently pretreated animals were i.n. infected with *C. muridarum,* or mock-infected. During the following 7 days body weight [%] (a) and clinical score (b) were assessed daily. On day 7 of challenge-infection, the surviving animals were sacrificed to determine the amount of viable, infectious chlamydia (IFU, c), and the levels of MPO (d), TNF-α (e) and IFN-γ (f) in their lung homogenate, see figure 5. Experimental setup, statistical analysis and meaning of the * are described above. LOD = limit of detection; ns = not significant.

### Intranasal vaccination with two-component 2cVAC is still effective but with a smaller degree pf protection as compared to 5cVAC in lung challenge infection with Ctr E.

In this modified "vaccination - *Ctr* E lung challenge experiment", vaccinated mice received only 2 (2cVAC) of the 5 components contained in 5cVAC plus adjuvant (triangles) by the i.n. route. Animals pretreated with [buffer plus adjuvant] (open grey squares), or [buffer alone] (black circles), served as negative controls. For better comparability, data obtained in a previous experiment with i.n. 5cVAC pretreatment are also depicted (triangles). Per group, n = 8-9 differently pretreated animals were i.n. infected with *Ctr* E*,* or mock-infected (open triangles). On each of the following days, body weight [%] (a) and clinical score (b) were assessed. On day 7 after i.n. challenge-infection, the surviving animals were sacrificed for determination of the amount of viable, infectious chlamydia (IFU, c), and the levels of MPO (d), TNF-α (e) and IFN-γ (f) in the lung homogenate, see figure 5. Experimental setup, the antigens contained in 2cVAC, statistical analysis and meaning of the * are described above. Statistical significance (p<0.05) between the 5cVAC and the adjuvant/buffer control groups is marked with # in the panels a, c, d, e and f. LOD = limit of detection; ns = not significant.

### IgA, IgM and total IgG antibody response profiles towards the 5cVAC antigen-mix or the single vaccine components in mouse plasma of 5 individual mice after vaccination with 5cVAC, 7 days before or after Ctr E challenge, respectively.

Moreover, less limiting anti-5cVac IgG of pool-plasma of n = 9 mice obtained 7 days p.i. was also analyzed. To get insight into individual antibody responses, microtiter plates were coated for an ELISA with 5cVAC or its single five protein components as antigens. To get an impression of individual immune responses before and after challenge infection, plasma of the first 5 mice which were i.n. vaccinated with 5cVAC was analyzed (18 upper panels). A small amount of blood was drawn for analysis 7 days before (and 7 days after) i.n. *Ctr* E infection. Thus, the plasma samples had to be diluted starting at 1:316 for IgA and IgM, or 1:3,160 for IgG, respectively. As secondary antibodies, enzyme-bound mAbs against mouse IgA (left row), IgM (middle row), or IgG (right row), see figure 7, respectively, were used. To permit direct comparison of the obtained results, the microtiter plates were coated with equimolar amounts of all individual recombinant antigens, and arbitrary units of the 3 different Ig's [U] were calculated in relation to a standard curve obtained with pool plasma from mice after 5cVAC application and *Ctr* E challenge. Negative controls with plasma of individual non-vaccinated Ctr E-challenged or non-challenged mice remained for <1 U (data not shown). The experimental setup of the mouse model can be found above.

Moreover, in less limited pool plasma of n = 9 5cVAC-treated and *Ctr* E challenged mice, total IgG against the 5cVAC mix and the individual recombinant antigens was also determined - this time starting with a dilution of 1:316 (right lower panel of figure 7). Pool plasma of non-vaccinated control mice which were i.n. challenged for 7 days with Ctr E served as negative control. Even only 1:100 diluted, its OD depending on total IgG against the 5cVAC mix remained <0.3. The broken vertical line at 1:3,160 indicates the lowest dilution used in the total IgG analysis of the plasma samples of the 5 individual mice.

The antibody responses against the different antigens varied largely from mouse to mouse. The highest responses were obtained against PmpD and Ctad1, and partially also against PmpH. Compared to the negative control, no antibody response against PmpA could be detected within the sensitivity of this assay.

As demonstrated, the immunogenic composition is useful as a vaccine against *Chlamydia* infection. In particular, combining the antigens with the adjuvant c-diAMP as representative of CDN demonstrates usefulness of the Vaccine according to the present invention. Especially mucosal administration is favorable.

## Claims

1. An immunogenic composition comprising at least three *Chlamydia ssp.* surface antigens selected from the group of polymorphic membrane protein PmpA, PmpD, PmpG and PmpH in combination with the antigen Ctad1.

2. The immunogenic composition according to claim 1 comprising at least PmpA, PmpD, PmpG, PmpH, Ctad1.

3. The immunogenic composition according to any one of the previous claims wherein the *Chlamydia ssp.* is *Chlamydia trachomatis.*

4. The immunogenic composition according to claim 3 wherein *Chlamydia trachomatis* is *Chlamydia trachomatis* serotype E.

5. The immunogenic composition according to any one of the previous claims further comprising an adjuvant.

6. The immunogenic composition according to claim 5 wherein the adjuvant is a compound according to formula (I) 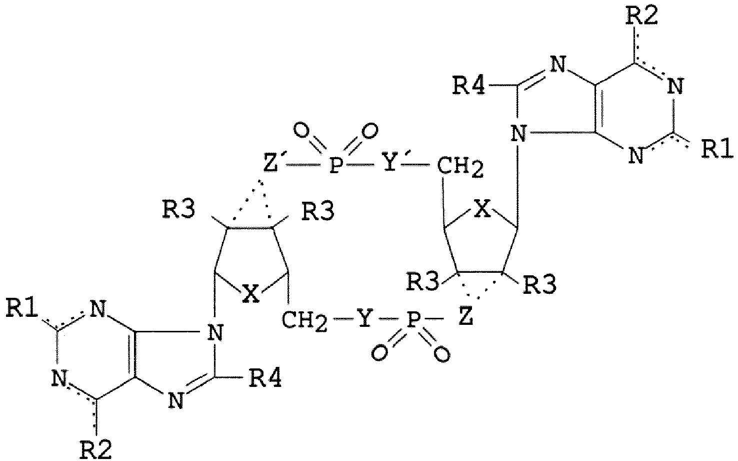 wherein
X is independently from one another S, N, O, CH₂;
Y, Y' is independently from one another NH, CH₂, O;
Z, Z' is independently from one another NH, CH₂, O;
R₁ is independently from one another hydrogen or O or absent;
R₂ is independently from one another NH₂, O, H, or a hydrogen;
R₃ is independently from one another absent if a covalent bond is present between the Z or Z' and the C atom, or is hydrogen, OH, halogen, a straight or branched C₁-C₆ alkyl group, or a straight or branched C₁-C₆ alkoxy group which may optionally be substituted;
R₄ is independently from one another hydrogen, halogen, or a straight or branched C₁-C₆ alkyl group which may optionally be substituted;
... is a single or double bond;
or conjugates thereof, and salts or solvates thereof.

7. The immunogenic composition according to claim 6 **characterized in that** in the compound according to formula I any one of the purine residue is an adenine, xanthine or hypoxanthine residue or combinations thereof, in particular, both purine residues being adenine.

8. The immunogenic composition for use according to any one of claims 6 or 7 **characterized in that** in formula I R3 is a OH group, X is an oxygen atom and Y, Y', Z and Z' are oxygen.

9. The immunogenic composition according to any one of the claims 6 to 8 **characterized in that** the compound according to formula I is a cyclic bis (3'-5') diodenylic acid (CDA) or c-diAMP, c-diIMP, c-IAMP, or a cyclic diAMP thiophosphate, c-diMP thiophosphate and c-IAMP thiophosphate.

10. The immunogenic composition according to any one of the preceding claims wherein the antigens are PmpA of SEQ ID No. 1, PmpD of SEQ ID No. 2, PmpG of SEQ ID No. 3, PmpH of SEQ ID No. 4, and Ctad1 of SEQ ID No. 5 derived from the respective proteins of *Chlamydia trachomatis* serotype E, PmpA of SEQ ID No. 6, PmpD of SEQ ID No. 7, PmpG of SEQ ID No. 8, PmpH of SEQ ID No. 9, and Ctad1 of SEQ ID No. 10 derived from the respective proteins of *Chlamydia trachomatis* serotype B, PmpA of SEQ ID No. 11, PmpD of SEQ ID No. 12, PmpG of SEQ ID No. 13, PmpH of SEQ ID No. 14, and Ctad1 of SEQ ID No. 5 1 derived from the respective proteins of *Chlamydia trachomatis* serotype LGV, PmpA of SEQ ID No. 16, PmpD of SEQ ID No. 17, PmpG of SEQ ID No. 18, PmpH of SEQ ID No. 19, and Ctad1 of SEQ ID No. 20 derived from the respective proteins of *C*. *muridarum.*

11. A vaccine comprising the immunogenic composition according to any one of the claims 1 to 10.

12. The vaccine according to claim 11 or the immunogenic composition according to any one of the claims 1 to 10 for use in eliciting an immune response against *Chlamydia ssp.,* or a component thereof, in an animal, preferably, wherein the animal is a human.

13. The vaccine according to claim 11 or 12 or the immunogenic composition according to any one of claims 1 to 9 for use in treating or preventing the infection by *Chlamydia ssp.* in an animal.

14. The vaccine according to any one of the claims 11 to 13 according to claim 11 or the immunogenic composition according to any one of the claims 1 to 10 wherein the *Chlamydia ssp.* is a *Chlamydia trachomatis* or a *Chlamydia muridarum.*

15. The vaccine according to any one of claims 11 to 14 or the immunogenic composition according to any one of the claims 1 to 10 wherein the use in the treatment or prevention of infection comprises mucosal administration of the vaccine, pharmaceutical composition or immunogenic composition preferably at least three times, in particular, intranasal application.
